# EUROPÄISCHE PATENTSCHRIFT

(11) **EP 3 761 195 B1**
(45) Veröffentlichungstag und Bekanntmachung des Hinweises auf die Patenterteilung: **07.06.2023**
(21) Anmeldenummer: 19186341.4
(22) Anmeldetag: 15.07.2019
(51) Int. Cl.: G06F 21/10, A62C 99/00

(54) **VERIFIZIERUNGSVERFAHREN FÜR EINE SAUERSTOFFREDUZIERUNGSANLAGE**
METHOD FOR VERIFYING AN OXYGEN REDUCTION PLANT
PROCÉDÉ DE VÉRIFICATION D'UNE INSTALLATION DE RÉDUCTION D'OXYGÈNE

(30) Priorität: 01.07.2019 DE 102019117651
(43) Veröffentlichungstag der Anmeldung: 06.01.2021
(73) Patentinhaber: Wagner Group GmbH, 30853 Langenhagen (DE)
(72) Erfinder: Beckers, Stefan, 31246 Ilsede (DE); Henkel, Andreas, 31177 Harsum (DE)
(74) Vertreter: Götz, Georg Alois

(56) Entgegenhaltungen:
- CN-A- 110 247 933
- US-A1- 2008 135 265
- US-A1- 2013 179 984
- US-A1- 2017 161 470

## Beschreibung

Die Erfindung betrifft ein Verifizierungsverfahren zur Verifizierung von im Betrieb einer Sauerstoffreduzierungsanlage genutzten Anlagenfunktionen der Sauerstoffreduzierungsanlage, wobei die Sauerstoffreduzierungsanlage zum Absenken und/oder Halten eines Sauerstoffkonzentrationsniveaus in mindestens einem Schutzbereich durch Zuführung von aus mindestens einer Inertgasquelle stammendem, insbesondere mittels mindestens eines Inertgaserzeugers erzeugtem Inertgas und zum Überwachen oder Anheben des, insbesondere zuvor abgesenkten, Sauerstoffkonzentrationsniveaus in dem Schutzbereich oder einem Überwachungsbereich ausgebildet ist.

Die Erfindung betrifft außerdem ein programmierbares Steuerungsmodul nach Anspruch 17 und eine Sauerstoffreduzierungsanlage mit einem solchen programmierbaren Steuerungsmodul nach Anspruch 19.

Zur Brandvorbeugung und -vermeidung werden in der Praxis oft Sauerstoffreduzierungsanlagen eingesetzt. Diese Anlagen ermöglichen es, den Sauerstoffgehalt innerhalb eines Schutzbereichs auf ein Niveau zu senken, das unterhalb des Sauerstoffgehalts in der Umgebungsluft und insbesondere unterhalb der Entzündungsgrenze von im Schutzbereich vorhandenen Materialien liegt. Als Schutzbereich wird im Allgemeinen ein räumlich abgegrenzter bzw. umschlossener Bereich verstanden, in dem die Sauerstoffkonzentration zur Brandvermeidung abgesenkt und innerhalb eines vorbestimmten Wertebereichs geregelt wird. Die Absenkung der Sauerstoffkonzentration erfolgt durch Zuführung von Inertgasen oder inertgasangereicherter Luft, insbesondere Stickstoff oder stickstoffangereicherter Luft, in den Schutzbereich. Damit wird das Verhältnis zwischen Inertgas oder inertgasangereicherter Luft und Sauerstoff so eingestellt, dass im Ergebnis der Sauerstoffgehalt der im Schutzbereich enthaltenen Luft reduziert wird. Vorzugsweise bleibt weiterhin ausreichend Sauerstoff vorhanden, sodass sich Personen in dem Schutzbereich aufhalten können. Neben dem Schutzbereich ist die Überwachung der Sauerstoffkonzentration oftmals aber auch in einem sogenannten Überwachungsbereich erforderlich. Ein Überwachungsbereich kann ein räumlich abgegrenzter bzw. umschlossener Bereich sein, insbesondere in für das Servicepersonal der Sauerstoffreduzierungsanlage oder sonstige Personen zugänglichen Räumen, die z. B. benachbart zu einem Schutzbereich liegen. Obwohl in einem Überwachungsbereich an sich keine Inertgas-Einleitung vorgesehen ist, könnte insbesondere durch Leckagen im Leitungssystem ungewollt eine Einleitung von Inertgasen erfolgen. Wird in einem Schutzbereich oder einem Überwachungsbereich ein zu niedriges Sauerstoffkonzentrationsniveau, aufgrund von entweder beabsichtigter (Schutzbereich) oder unbeabsichtigter (Überwachungsbereich) Einleitung von Inertgas, lässt sich die Sauerstoffkonzentration aber auch mittels der Sauerstoffreduzierungsanlage anheben.

Üblicherweise ist zur Regelung der einzelnen Komponenten der Sauerstoffreduzierungsanlage eine Steuerzentrale vorgesehen, die z. B. einerseits die Erzeugung des Inertgases anforderungsgerecht regelt und andererseits mit Sauerstoffkonzentrationssensoren verbunden ist, um die entsprechenden Inertagsmengenanforderungen an die Inertgasquellen, insbesondere Inertgaserzeuger weiterzugeben. Neben den üblichen, stationären Inertgaserzeugern, wie z. B. einem Membranstickstofferzeuger oder einem Druckwechseladsorptionsstickstofferzeuger kommen als Inertgasquellen aber auch insbesondere mobile Inertgaserzeuger sowie mobile Tanklastwagen oder stationäre Inertgastanks, in Form von größeren Behälter oder auch Inertgasflaschen bzw. - flaschenbatterien in Frage. Darüber hinaus ist die Steuerzentrale mit mehreren Aktoren gekoppelt, um eine Verteilung des erzeugten Inertgases zu steuern. Die Steuerzentrale kann mehrere Schutzbereiche getrennt voneinander regeln, wobei jedem Schutzbereich ein oder mehrere Inertgasquellen, insbesondere Inertgaserzeuger zugeordnet sind und jeder Schutzbereich mit einem oder mehreren Sauerstoffkonzentrationssensoren ausgestattet ist.

Aus der zeitrangälteren PCT/EP2019/061910 ist ein dezentrales Steuerungs- und Regelungssystem einer solchen Sauerstoffreduzierungsanlage bekannt, das mehrere miteinander signalverbundene Reglermodule zur Ausführung von Regelungsfunktionen aufweist, wobei die Regelungsfunktionen dezentral auf den Reglermodulen verteilt sind. Gemäß der PCT/EP2019/061910 sind die Begriffe "Reglermodul" und "Regelungsfunktion" als Kurzformen für die Begriffe "Steuer- und Reglermodul" sowie "Steuerungs- und Regelungsfunktion" zu verstehen. Eingesetzt werden standardisierte und hardwareseitig weitgehend identische Reglermodule, denen unterschiedliche Funktionen zum Betrieb der Sauerstoffreduzierungsanlage zugewiesen werden können. So kann einem Reglermodul als Prozessregler die Steuerung und/oder Regelung der Inertgaserzeugung, einem weiteren Reglermodul als Bereichsregler die Überwachung eines Sauerstoffkonzentrationsniveaus in einem Schutzbereich oder Überwachungsbereich und schließlich einem Reglermodul als Masterregler eine kommunikative Abstimmung zwischen den weiteren Reglermodulen zugewiesen werden. Die Reglermodule sind über ein Bussystem miteinander verbunden und bereits im Grundausbau des Steuerungs- und Regelungssystems vorhanden sowie zur Ausführung grundlegender Kern- oder Basisfunktionen, die für den Betrieb der Sauerstoffreduzierungsanlage zwingend erforderlich sind, konfiguriert. Zur Ausführung weiterer, optionaler Regelungsfunktionen können die Reglermodule mit zusätzlichen Baugruppen und/oder Steckkarten erweitert werden. Nachteilig ist hierbei jedoch, dass das Steuerungs- und Regelungssystem sowie die Funktionen im Betrieb der Sauerstoffreduzierungsanlage auch nach Auslieferung, Installation und Inbetriebnahme erweiterbar sind, ohne, dass aus technischen Gründen eine Prüfung oder Freigabe im Hinblick auf sicherheitsrelevante Aspekte erforderlich ist. Beispielsweise könnten ungeprüfte Baugruppen und/oder Steckkarten verwendet werden.

Es ist auch bekannt, derartige Reglermodule als speicherprogrammierbare Steuerung (SPS) auszugestalten. Eine speicherprogrammierbare Steuerung weist mehrere Eingänge und Ausgänge auch als Eingangskanäle bzw. Ausgangskanäle bezeichnet auf, die an Sensoren und Aktoren der Anlage angebunden sind. Über eine Schnittstelle wird ein Anwenderprogramm geladen, das u. a. festlegt, wie die Ausgänge in Abhängigkeit von den Eingängen geschaltet werden sollen und so die Anlage über die angebundenen Sensoren und Aktoren steuert und/oder regelt.

Im Bereich von Computersoftware ist es üblich, lizenzpflichtige Software mit einem Kopierschutz auszustatten. Mit der Software wird zum Beispiel ein Kopierschutzstecker, ein sogenannter Dongle, ausgeliefert, der auf eine Schnittstelle des Computers, z.B. einen USB-Anschluss, aufgesteckt wird. Die geschützte Software überprüft bei Benutzung, ob der Kopierschutzstecker vorhanden ist, ist dies nicht der Fall können beispielsweise nur noch eingeschränkte

### In Erwiderung auf den erweiteren europäischen Recherchenbericht (Reinschrift)

Programmfunktionen freigegeben oder die Benutzung der Software verweigert werden. Andere Formen von Hardware-Dongles sind insbesondere die Verwendung von Public-Private-Key-Verschlüsselungsverfahren, bei denen die verschlüsselten Dongle-Informationen im Flash-Rom des Computers hinterlegt sein können. Auch Netzwerkvarianten eines Dongle-Kopierschutzes sind bekannt.

Aus der US 2008/135265 A1 sind ein Verfahren sowie eine Vorrichtung zum geregelten Zuführen von Zuluft über einen ersten Inertgasvolumenstrom und zusätzlich einen zweiten Frischgasvolumenstrom in einen dauerinertisierten Raum bekannt. Über zumindest eine Steuereinheit wird eine erste Volumenstromrate, mit welcher das von der Inertgasquelle bereitgestellte Inertgas in die Raumluftatmosphäre des dauerinertisierten Raums zugeführt wird, in Abhängigkeit vom Inertisierungsniveau des Raums geregelt. Die Überwachung der Raumluftatmosphäre an einer oder mehreren Stellen innerhalb des dauerinertisierten Raums mit jeweiligen Sensoren umgesetzt.

Ein Verfahren zum Lizenz-Sharing in einem konventionellen Telekommunikationsnetzwerk ist in der US 2017/161470 A1 offenbart, wobei eine Lizenz grundsätzlich die Nutzung einer Software autorisieren soll. Drei erste und zweite Geräte verwenden in einer Netzwerkarchitektur nicht-statistische Lizenz-Ressourcen der Typen a, b und c. Ein Lizenzserver vergleicht die ermittelte Anzahl insgesamt genutzter Lizenz-Ressourcen für jeden Typ mit einer jeweiligen Alarmschwelle. Die Alarmschwelle kann als prozentualer oder als konkreter numerischer Werte hinterlegt sein.

Es ist die Aufgabe der vorliegenden Erfindung, die Sicherheit beim Betrieb einer Sauerstoffreduzierungsanlage zu verbessern, insbesondere ein Verfahren bereitzustellen, um im Betrieb einer Sauerstoffreduzierungsanlage genutzten Anlagenfunktionen zu prüfen und sicherzustellen, dass ausschließlich zugelassene, bzw. freigegebene Anlagenfunktionen genutzt werden.

Die Aufgabe wird gelöst durch ein Verifizierungsverfahren nach Anspruch 1, durch ein programmierbares Steuerungsmodul nach Anspruch 17 sowie durch eine Sauerstoffreduzierungsanlage nach Anspruch 19

Ein erfindungsgemäßes Verifizierungsverfahren für eine Sauerstoffreduzierungsanlage der eingangs beschriebenen Art weist die nachfolgenden Verfahrensschritte auf:
- Erfassen von im Betrieb einer Sauerstoffreduzierungsanlage genutzten Anlagenfunktionen,
- Erstellen eines Lizenzdatensatzes, wobei der Lizenzdatensatz von den genutzten Anlagenfunktionen verwendete Lizenzen (l_{1y}, l_{2y} ..., l_{ny}) enthält,
- Einlesen eines auf einem Speichermedium bereitgestellten Verifizierungsdatensatzes, wobei der Verifizierungsdatensatz mindestens eine vorhandene Lizenz (L₁ₓ, L₂ₓ ... Lₙₓ) enthält,
- Feststellen einer Lizenzverletzung, sofern nicht jede verwendete Lizenz (l_{1y}, l_{2y}, ..., l_{ny}) von dem Verifizierungsdatensatz umfasst ist,
- Ausgeben einer Meldung, insbesondere einer Fehlermeldung und/oder Abschalten mindestens einer, insbesondere aller Anlagenfunktionen, sofern eine Lizenzverletzung erkannt wird.

Erfindungsgemäß wird das Verifizierungsverfahren somit während des Betriebs der Sauerstoffreduzierungsanlage, d. h. während die Sauerstoffreduzierungsanlage für ihren Betrieb erforderliche Anlagenfunktionen nutzt, durchgeführt und läuft im Hintergrund, während gleichzeitig die genutzten Anlagenfunktionen von einem Steuerungs- und Regelungssystem gesteuert und geregelt werden. Grundsätzlich werden Anlagenfunktionen der Sauerstoffreduzierungsanlage in Basisanlagenfunktionen, die für die Funktionsfähigkeit zwingend erforderlich sind und optionale Anlagenfunktionen, die je nach Bedarf, wahlweise zusätzlich zu den Basisanlagenfunktionen genutzt werden können, unterschieden. Insbesondere die Erzeugung einer zum Absenken und/oder Halten der Sauerstoffkonzentration in einem Schutzbereich benötigten Menge an Inertgas und das Freisetzen der erzeugten Menge an Inertgas in den entsprechenden Schutzbereich, aber auch die Überwachung des Schutzbereiches oder ein Anheben einer in dem Schutzbereich zuvor abgesenkten Sauerstoffkonzentration sind Basisanlagenfunktionen. Optionale Anlagenfunktionen sind z.B. die Verteilung des erzeugten Inertgases in mehrere Schutzbereiche, die rein vorsorgliche Überwachung der Sauerstoffkonzentration in Überwachungsbereichen, wie Nachbar-, Technik-, Maschinen- und Betriebsräumen, Überwachung von Umgebungsbedingungen, Luftumwälzung in Schutzbereichen und Überwachungsbereichen usw.

In einem, insbesondere ersten, Verfahrensschritt des erfindungsgemäßen Verifizierungsverfahrens werden im Betrieb und zum derzeitigen Zeitpunkt genutzte Anlagenfunktionen erfasst. Vorteilhaft hat sich erwiesen, hierbei alle genutzten Anlagenfunktionen zu erfassen, alternativ ist aber auch denkbar z. B. nur die optionalen Anlagenfunktionen zu erfassen.

In einem zweiten Verfahrensschritt, der insbesondere auf den ersten Verfahrensschritt folgt, wird ein Lizenzdatensatz erstellt. Der Lizenzdatensatz enthält von den genutzten Anlagenfunktionen verwendete Lizenzen. Hierzu kann weiter in lizenzpflichtige und lizenzfreie Anlagenfunktionen unterschieden werden. In der Regel sind Basisanlagenfunktionen lizenzfrei oder in einer Basislizenz zusammengefasst, optionale Anlagenfunktionen sind zumeist lizenzpflichtig. Vorzugsweise werden alle genutzten Anlagenfunktionen erfasst, alternativ ist aber auch denkbar nur die optionalen Anlagenfunktionen zu erfassen. In vorteilhafter Ausgestaltung wird der Lizenzdatensatz anhand aller von der Sauerstoffreduzierungsanlage genutzten Anlagenfunktionen erstellt und enthält die verwendeten Lizenzen aller lizenzpflichtiger Anlagenfunktionen.

In einem dritten Verfahrensschritt, der insbesondere auf den zweiten Verfahrensschritt folgt oder aber gleichzeitig mit dem ersten oder dem zweiten Verfahrensschritt durchgeführt wird, wird ein Datensatz, ein Verifizierungsdatensatz von einem Speichermedium eingelesen. Der Verifizierungsdatensatz enthält eine oder mehrere vorhandene Lizenzen, die vorzugsweise ebenfalls jeweils einer konkreten Anlagenfunktion oder, insbesondere in Form einer Basislizenz, mehreren (Basis-) Anlagenfunktionen zugeordnet sind.

In einem vierten Verfahrensschritt, der insbesondere auf den dritten Verfahrensschritt folgt, wird festgestellt, ob eine Lizenzverletzung vorliegt. Hierzu werden der Lizenzdatensatz und der Verifizierungsdatensatz miteinander abgeglichen, indem geprüft wird, ob die den Anlagenfunktionen zugeordneten jeweiligen verwendeten Lizenzen von dem Verifizierungsdatensatz umfasst sind, d. h. insbesondere, ob jeder verwendeten Lizenz des Lizenzdatensatzes eine entsprechende, vorhandene Lizenz des Verifizierungsdatensatzes zugeordnet werden kann. Sofern mindestens eine verwendete Lizenz nicht von dem Verifizierungsdatensatz umfasst ist, d. h. insbesondere mindestens einer verwendeten Lizenz keine entsprechende, vorhandene Lizenz zugeordnet werden kann, wird eine Lizenzverletzung festgestellt.

In einem fünften Verfahrensschritt, der insbesondere auf den vierten Verfahrensschritt folgt, wird schließlich eine Meldung, insbesondere eine Fehlermeldung ausgegeben, sofern eine Lizenzverletzung festgestellt wird. Eine Meldung kann grundsätzlich jegliche Art von im Verifizierungsverfahren ausgegebener Meldung sein, anhand der eine Lizenzverletzung, durch einen Benutzer oder das Servicepersonal erkennbar ist. In der Regel ist eine Fehlermeldung im System intern hinterlegt und nur durch Abfrage der entsprechenden Parameter zu erkennen. Eine Störmeldung ist hingegen eine spezielle Fehlermeldung, die mit dem Zweck ausgegeben wird, einen Benutzer, insbesondere das Servicepersonal aktiv, z. B. auch durch optische oder akustische Signale, auf das Vorliegen einer Lizenzverletzung aufmerksam zu machen. Bei Ausgabe einer Meldung, insbesondere einer Fehlermeldung oder Störmeldung, ist grundsätzlich vorgesehen, dass die Sauerstoffreduzierungsanlage ihren normalen Betrieb fortsetzt, ohne Eingriff in die oder Beeinträchtigung der genutzten Anlagenfunktion.

Zusätzlich oder alternativ kann oder können bei Feststellen einer Lizenzverletzung außerdem mindestens eine, insbesondere lizenzpflichtige, oder auch alle genutzten Anlagenfunktionen abgeschaltet werden.

Sofern eine Lizenzverletzung nicht festgestellt wird, wird ebenfalls der normale Betrieb der Sauerstoffreduzierungsanlage fortgesetzt und die derzeit genutzten Anlagenfunktionen weiterhin ausgeführt.

Das Verifizierungsverfahren kann einmalig, z.B. zur Freigabe der Sauerstoffreduzierungsanlage nach Installation und bei Inbetriebnahme ausgeführt werden oder in bestimmten oder regelmäßigen zeitlichen Abständen wiederholt werden, um eine dynamische Erweiterung der Anlagenfunktionen während des Betriebs und nach der Inbetriebnahme zu prüfen und/oder zu verifizieren.

Vorteilhafte Ausführungsformen sind in den Unteransprüchen beansprucht und werden nachfolgend näher erläutert.

Zur Anbindung an die Sauerstoffreduzierungsanlage ist das Verifizierungsverfahren erfindungsgemäß auf mindestens einem programmierbaren Steuerungsmodul (in Fachkreisen auch kurz als "Regler" oder "Reglermodul" bezeichnet) implementiert, wobei die genutzten Anlagenfunktionen anhand einer spezifischen Belegung von Signaleingangs- und Signalausgangskanälen des mindestens einen Steuerungsmoduls erfasst werden.

Vorzugsweise wird das Verifizierungsverfahren in einem programmierbaren Steuerungsmodul implementiert, das auch für die Steuerung und Regelung der Sauerstoffreduzierungsanlage konfiguriert ist. Bei dem programmierbaren Steuerungsmodul kann es sich bspw. um eine speicherprogrammierbare Steuerung handeln, deren Eingangs- und Ausgangskanäle zur Steuerung und Regelung der Sauerstoffreduzierungsanlage mit entsprechenden Anlagenfunktionen belegt sind, d. h. an die zur Durchführung dieser Anlagenfunktionen erforderlichen Sensoren und Aktoren angebunden. Das Verifizierungsverfahren kann insbesondere in Form einer Programmierung in einem Speicherkern des Steuerungsmoduls hinterlegt sein, wobei die genutzten Anlagenfunktionen anhand der Belegung der Eingangs- und Ausgangskanäle erfasst werden können. Hierbei ist von besonderem Vorteil, dass das Verifizierungsverfahren auch in ein Steuer- und Regelungssystem einer bereits im Betrieb befindlichen Sauerstoffreduzierungsanlage unkompliziert nachgerüstet werden kann.

In vorteilhafter Ausgestaltung des erfindungsgemäßen Verifizierungsverfahrens umfasst jede verwendete Lizenz (l_{1y}, l_{2y}, ..., l_{ny}) des Lizenzdatensatzes mindestens zwei Parameter, einen jeweiligen Lizenztyp (1, 2, ..., n) und eine dem jeweiligen Lizenztyp (1, 2, ..., n) zugeordnete erste Lizenzanzahl (yₗ₁, yₗ₂, ... yₗₙ) und jede vorhandene Lizenz (L₁ₓ, L₂ₓ ... Lₙₓ) des Verifizierungsdatensatzes mindestens zwei Parameter, einen jeweiligen Lizenztyp (1, 2, ..., n) und eine dem jeweiligen Lizenztyp (1, 2, ..., n) zugeordnete bzw. zweite Lizenzanzahl (x_{L1}, x_{L2}, ... x_{Ln}), mit den Variablen y = 0, 1, ..., n für die erste Lizenzanzahl und x = 0, 1, ..., n für die die zweite Lizenzanzahl. Zum Feststellen einer Lizenzverletzung erfolgt eine, insbesondere matrixartige, d.h. reihen- oder spaltenweise Zuordnung der verwendeten Lizenzen (l_{1y}, l_{2y}, ..., l_{ny}) zu den vorhandenen Lizenzen (L₁ₓ, L₂ₓ ... Lₙₓ).

Als Lizenztypen werden die konkreten Anlagenfunktionen zugeordneten Lizenzen unterschieden. Bspw. kann ein Lizenztyp "Luftumwälzung" der insbesondere optionalen Anlagenfunktionen Luftumwälzung zugeordnet sein, wohingegen ein Lizenztyp "Basislizenz" mehreren zum Betrieb der Sauerstoffreduzierungsanlage zwingend erforderlichen Basisanlagenfunktionen zugeordnet sein kann, wie z. B. der Erzeugung einer zum Absenken und/oder Halten der Sauerstoffkonzentration in einem Schutzbereich benötigten Menge an Inertgas, der Freisetzung der erzeugten Menge an Inertgas in den entsprechenden Schutzbereich und/oder dem Anheben der in dem Schutzbereich zuvor abgesenkten Sauerstoffkonzentration.

In Weiterbildung dieser Ausgestaltung wird zum Feststellen einer Lizenzverletzung mindestens ein Lizenzwert (z₁, z₂, ..., zₙ), mit der Variablen z = -n, ..., -1, 0, 1, ..., n, jeweils einander, insbesondere reihen- oder spaltenweise, zugeordneter Lizenztypen (1, 2, ..., n) ermittelt, indem die erste Lizenzanzahl (yₗ₁, yₗ₂, ... yₗₙ) der verwendeten Lizenzen (l_{1y}, l_{2y}, ..., l_{ny}) von der zweiten Lizenzanzahl (x_{L1}, x_{L2}, ... x_{Ln}) der vorhandenen Lizenzen (L₁ₓ, L₂ₓ ... Lₙₓ) subtrahiert wird, und wobei eine Lizenzverletzung festgestellt wird, sofern einer der ermittelten Lizenzwerte (z₁, z₂, ..., zₙ) < 0 ist.

Als vorteilhafte Option können einem Benutzer oder dem Servicepersonal der Sauerstoffreduzierungsanlage die ermittelten Lizenzwerte (z₁, z₂, ..., zₙ) z.B. in Form einer optischen Darstellung auf einem Bedien- und Anzeigepanel oder einem Bildschirm ausgegeben werden. Anhand der ermittelten Lizenzwerte (z₁, z₂, ..., zₙ) > 0 kann die jeweilige Anzahl derzeit verfügbarer Lizenzen festgestellt werden und der Benutzer oder das Servicepersonal wird über frei zur Verfügung stehende, d.h. nicht verwendete, jedoch vorhandene Lizenzen informiert. Auf diese Weise können für zugeordnete Anlagenfunktionen vorhandene Lizenzen übersichtlich und effizient verwaltet werden. Ein Lizenzwert z = 0 gibt an, dass alle vorhandenen Lizenzen von entsprechenden Anlagenfunktionen genutzt werden bzw. alle verwendeten Lizenzen durch den Verifizierungsdatensatz verifiziert sind. Selbstverständlich ist es umgekehrt ebenso denkbar die zweite Lizenzanzahl (x_{L1}, x_{L2}, ... x_{Ln}) der vorhandenen Lizenzen (L₁ₓ, L₂ₓ ... Lₙₓ) von der ersten Lizenzanzahl (yₗ₁, yₗ₂, ... yₗₙ) der verwendeten Lizenzen (l_{1y}, l_{2y}, ..., l_{ny}) zu subtrahieren, wobei entsprechend eine Lizenzverletzung festgestellt wird, sofern einer der so ermittelten Lizenzwerte (z₁, z₂, ..., zₙ) > 0 ist.

Nach einer optionalen Verfahrensvariante wird eine Prüfung, ob der Verifizierungsdatensatz einen Freischaltcode enthält durchgeführt, wobei eine Meldung, insbesondere eine Störmeldung, ausgegeben wird und/oder mindestens eine, vorzugsweise alle, Anlagenfunktion abgeschaltet werden, sofern der Freischaltcode nicht erkannt wird.

Durch den Freischaltcode kann somit eine zusätzliche und ggf. übergeordnete Sicherheitsabfrage vorgesehen sein, die insbesondere vor, während oder nach dem Einlesen des Verifizierungsdatensatzes durchgeführt werden kann und so z. B. sicherstellt, dass das Speichermedium den richtigen, für die zugehörige Sauerstoffreduzierungsanlage vorgesehenen Verifizierungsdatensatz enthält.

Eine ähnliche Sicherheitsabfrage kann in bevorzugter Ausgestaltung durch das Einlesen des Verifizierungsdatensatzes über eine mit dem Speichermedium signalübertragend verbundene oder verbindbare Schnittstelle erfolgen, wobei eine Meldung, insbesondere eine Störmeldung, ausgegeben wird und/oder mindestens eine, vorzugsweise alle, Anlagenfunktion abgeschaltet werden, sofern die signalübertragende Verbindung zwischen dem Speichermedium und der Schnittstelle nicht erkannt wird.

Auch wenn grundsätzlich denkbar ist, dass die Schnittstelle intern, in das programmierbare Steuerungsmodul integriert ist und das Speichermodul z. B. als interner Speicherkern ausgeführt ist, so ist es nach einer Weiterbildung der obigen Verfahrensvariante vorteilhaft, die Schnittstelle als externe Schnittstelle, insbesondere als USB-Anschluss, und das Speichermodul als externes Speichermodul, insbesondere als USB-Dongle, auszubilden, wobei das externe Speichermodul dann manuell von einem Benutzer mit der externen Schnittstelle verbindbar ist.

Vorzugsweise wird das Verifizierungsverfahren gemäß einer Erfindungsausgestaltung in bestimmten oder regelmäßigen zeitlichen Abständen wiederholt, und gleichzeitig bei Ausgabe der Meldung, insbesondere der Fehlermeldung, ein Zeitmesser gestartet, um die Zeitspanne während der die Meldung, insbesondere die Fehlermeldung, ausgegeben wird zu erfassen. Der Zeitmesser kann hierzu die Zeitspanne vorwärts oder rückwärts messend konfiguriert sein kann.

Sofern bei einem erneuten Durchlauf des Verfizierungsverfahrens eine Lizenzverletzung nicht länger festgestellt wird, ist vorzugsweise eine automatische Rücksetzung des Zeitmessers sowie der Fehler- oder Störmeldung vorgesehen. Alternativ oder zusätzlich ist auch denkbar, dass der Zeitmesser sowie die Fehler- oder Störmeldung durch einen Benutzer oder das Servicepersonal der Sauerstoffreduzierungsanlage manuell zurückgesetzt werden kann.

In Weiterbildung dieser Erfindungsausgestaltung wird mit Erreichen oder nach Ablauf einer vorgegebenen Zeitspanne, während der die Meldung, insbesondere die Fehlermeldung, ausgegeben wird, eine Störmeldung ausgegeben und/oder mindestens eine Anlagenfunktion, insbesondere alle Anlagenfunktionen, abgeschaltet.

Nach einer besonders vorteilhaften Weiterbildung dieser Erfindungsausgestaltung kann mit Erreichen oder nach Ablauf einer vorgegebenen Zeitspanne, während der die Meldung, insbesondere eine Fehlermeldung und/oder eine Störmeldung ausgegeben wird ein Abschalten mindestens einer, insbesondere aller Anlagenfunktionen, der Sauerstoffreduzierungsanlage erfolgen.

Konkret kann z.B. mit Erreichen oder nach Ablauf einer ersten vorgegebenen Zeitspanne, insbesondere nach 60 Minuten, während der eine Fehlermeldung ununterbrochen ausgegeben wurde bzw. eine Lizenzverletzung ununterbrochen festgestellt wurde, anstelle der Fehlermeldung eine Störmeldung ausgegeben werden. Mit Erreichen oder nach Ablauf einer zweiten vorgegebenen Zeitspanne, z.B. nach weiteren 72 Stunden, während der die Störmeldung ununterbrochen ausgegeben wurde bzw. eine Lizenzverletzung ununterbrochen festgestellt wurde, kann dann eine Abschaltung mindestens einer, vorzugsweise aller, Anlagenfunktionen erfolgen.

Für die Bedienung und den Betrieb der Sauerstoffreduzierungsanlage haben sich die vorgegebenen Zeitspannen als vorteilhaft erwiesen. So kann beispielsweise innerhalb der ersten vorgegebenen Zeitspanne von 60 Minuten einerseits das den Verifizierungsdatensatz enthaltende Speichermedium zum Austausch oder zum Aufspielen von Updates und/oder zusätzlichen, vorhandenen Lizenzen temporär entfernt werden, ohne dass in den Betrieb der Sauerstoffreduzierungsanlage eingegriffen würde. Die mit der zweiten Zeitspanne vorgegebenen 72 Stunden, stellen andererseits einen ausreichenden Zeitraum zur Verfügung eine ggf. unbeabsichtigte Lizenzverletzung zu beheben und/oder bei einer bevorstehenden Abschaltung ausgewählter oder aller Anlagenfunktionen erforderliche Vorbereitungen zu treffen, um eine mögliche Gefährdung durch die nicht länger genutzten Anlagenfunktionen zu vermeiden.

Besonders zweckmäßig ist eine Verfahrensvariante, bei der das Verifizierungsverfahren auf mehreren programmierbaren und miteinander signalverbundenen Steuerungsmodulen implementiert ist, wobei mindestens ein Steuerungsmodul als Bereichssteuerungsmodul mindestens einem Schutzbereich oder Überwachungsbereich zugeordnet ist und/oder mindestens ein Steuerungsmodul als Prozesssteuerungsmodul mindestens einer Inertgasquelle, insbesondere einem Inertgaserzeuger zugeordnet ist und/oder mindestens ein Steuerungsmodul als Mastersteuerungsmodul vorgesehen ist. Denkbar ist auch zwei oder mehr Mastersteuerungsmodule zur Schaffung einer entsprechenden Redundanz vorzusehen.

Gerade zur Nachrüstung eines eingangs beschriebenen, dezentralen Steuerungs- und Regelungssystem einer Sauerstoffreduzierungsanlage ist es vorteilhaft, auch das erfindungsgemäße Verifizierungsverfahren dezentral auf mehreren programmierbaren Steuerungsmodulen, die insbesondere über ein Bussystem oder Ringbussystem oder auch ein (drahtloses) Netzwerk miteinander signalverbunden sind, vorzugsweise auf einem jeweiligen Speicherkern, zu implementieren. Vorteilhaft ist, wenn die mehreren programmierbaren Steuerungsmodule weitestgehend identisch aufgebaut sind, jedoch spezifischen Anlagenteilen der Sauerstoffreduzierungsanlage, insbesondere auch örtlich, zugeordnet und zur Steuerung und Regelung der dort lokal genutzten Anlagenfunktionen konfiguriert sind. So kann beispielsweise mindestens ein Bereichssteuerungsmodul einem oder mehreren Schutzbereichen und/oder Überwachungsbereichen zugeordnet und u. a. zur Überwachung und Regelung der dortigen Sauerstoffkonzentration konfiguriert sein, mindestens ein Prozesssteuerungsmodul einer oder mehreren Inertgasquellen, insbesondere einem oder mehreren Inertgaserzeugern zugeordnet und zur Steuerung der erzeugten Inertgasmengen konfiguriert sein und mindestens ein Mastersteuerungsmodul zur kommunikativen Abstimmung der übrigen Steuerungsmodule konfiguriert sein.

Um alle lokal genutzten Anlagenfunktion erfassen zu können hat es sich in Weiterbildung als vorteilhaft erwiesen, dass das Erfassen genutzter Anlagenfunktionen, insbesondere lokal, auf dem mindestens einen Bereichssteuerungsmodul und/oder dem mindestens einen Prozesssteuerungsmodul und/oder dem mindestens einen Mastersteuerungsmodul implementiert ist, wobei die dem jeweiligen Steuerungsmodul lokal zugeordneten Anlagenfunktionen anhand einer spezifischen Belegung von Signaleingangs- und Signalausgangskanälen des jeweiligen Bereichssteuerungsmoduls und/oder Prozesssteuerungsmoduls und/oder Mastersteuerungsmoduls erfasst werden können.

Ebenso ist es vorteilhaft, dass auch das Erstellen des Lizenzdatensatzes lokal auf dem jeweiligen Steuerungsmodul implementiert ist. Bevorzugt zeichnet sich das Verfahren daher durch lokales Erstellen mindestens eines Lizenzdatensatzes aus, der von dem jeweiligen Steuerungsmodul verwendete Lizenzen (l_{1y}, l_{2y} ..., l_{ny}) enthält, wobei jede dort verwendete Lizenz (l_{1y}, l_{2y} ..., l_{ny}) mindestens einer lokal genutzten Anlagenfunktionen zugeordnet ist und mindestens zwei Parameter, einen jeweiligen Lizenztyp (1, 2, ..., n) und eine dem jeweiligen Lizenztyp (1, 2, ..., n) zugeordnete erste Lizenzanzahl (yₗ₁, yₗ₂, ... yₗₙ) umfasst, mit der jeweiligen Variablen y=1,2,...,n.

Insbesondere bei Konfiguration eines Steuerungsmoduls als Mastersteuerungsmodul zur Überwachung der Kommunikation hat es sich als vorteilhaft herausgestellt, dass ein Erstellen eines globalen Lizenzdatensatzes auf dem mindestens einen Mastersteuerungsmodul implementiert ist, und der globale Lizenzdatensatz durch Zusammenfügen der lokal erstellten Lizenzdatensätze gebildet wird, indem die ersten Lizenzanzahlen (yₗ₁, yₗ₂, ... yₗₙ) der von den jeweiligen Steuerungsmodulen lokal verwendeten Lizenzen (l_{1y}, l_{2y}, ..., l_{ny}) jeweils einander zugeordneter Lizenztypen (1, 2, ..., n) addiert werden und wobei den von den jeweiligen Steuerungsmodulen lokal verwendeten Lizenzen (l_{11y}, l_{22y} ..., l_{nmy}) ein dem jeweiligen Steuerungsmodul entsprechender Herkunftsindikator (1, 2, ..., m) zugewiesen wird, mit der jeweiligen Variablen y = 1, 2, ..., n.

Der globale Lizenzdatensatz enthält somit nicht nur die Information über die Anzahl der insgesamt verwendeten Lizenzen der jeweiligen Lizenztypen, darüber hinaus ist auch die jeweilige Herkunft der verwendeten Lizenz enthalten, d. h. welches programmierbare Steuerungsmodul diese Lizenzen verwendet.

Bei der Implementierung des Verfizierungsverfahrens auf mehreren Steuerungsmodulen ist es gemäß Ausgestaltung zweckmäßig, dass das Einlesen des Verifizierungsdatensatzes und vorzugsweise auch das Feststellen einer Lizenzverletzung, insbesondere anhand des globalen Lizenzdatensatzes, auf dem mindestens einen Mastersteuerungsmodul implementiert ist.

Eine weitere, zusätzliche Sicherheitsabfrage kann nach einer Verfahrensvariante vorgesehen sein, indem der Verifizierungsdatensatz mindestens eine vorhandene Steuerungsmodullizenz (SL_{BX}, SL_{PX}, SL_{MX}) enthält und der Lizenzdatensatz von den jeweiligen Steuerungsmodulen verwendete Steuerungsmodullizenzen (sl_{By}, sl_{Py}, sl_{My}) enthält, wobei eine Lizenzverletzung festgestellt wird, sofern nicht jede verwendete Steuerungsmodullizenz (sl_{By}, sl_{Py}, sl_{My}) von dem Verifizierungsdatensatz umfasst ist.

Konkret umfasst in Weiterbildung dieser Verfahrensvariante, entsprechend der eingangs beschriebenen Feststellung einer Lizenzverletzung jede verwendete Steuerungsmodullizenz (sl_{By}, sl_{Py}, sl_{My}) des Lizenzdatensatzes mindestens zwei Parameter, einen jeweiligen Steuerungsmodultyp (B, P, M) und eine dem jeweiligen Steuerungsmodultyp (B, P, M) zugeordnete erste Steuerungsmodullizenzanzahl (y_{slB}, y_{slP}, y_{slM}) umfasst und jede vorhandene Steuerungsmodullizenz (SL_{Bx}, SL_{PX}, SL_{MX}) des Verifizierungsdatensatzes mindestens zwei Parameter, einen jeweiligen Steuerungsmodultyp (B, P, M) und eine dem jeweiligen Steuerungsmodultyp (B, P, M) zugeordnete zweite Steuerungsmodullizenzanzahl (x_{SLB}, x_{SLP}, x_{SLM}) umfasst und zum Feststellen einer Lizenzverletzung mindestens ein Steuerungsmodullizenzwert (z_{SL1}, z_{SL2}, ..., z_{SLn}) jeweils einander zugeordneter Steuerungsmodulindikatoren (B, P, M) ermittelt wird, indem die erste Steuerungsmodullizenzanzahl (y_{slB}, y_{slP}, y_{slM}) der verwendeten Steuerungsmodullizenzen (sl_{By}, sl_{Py}, sl_{my}) von der zweiten Steuerungsmodullizenzanzahl (x_{SLB}, x_{SLP}, x_{SLM}) der vorhandenen Steuerungsmodullizenzen (SL_{BX}, SL_{PX}, SL_{Mx}) subtrahiert wird, und wobei eine Lizenzverletzung festgestellt wird, sofern einer der ermittelten Steuerungsmodullizenzwerte (z_{SL1}, z_{SL2}, ..., z_{SLn}) < 0 ist, mit den jeweiligen Variablen y = 0, 1, ..., n und z = -n, -1, ..., 0, 1, n.

Schließlich ist gemäß einer vorteilhaften Ausgestaltung des erfindungsgemäßen Verifizierungsverfahrens mindestens eine hinterlegte Anlagenfunktion von einem Benutzer über eine Benutzeroberfläche manuell auswählbar. Hierbei ist es vorteilhaft, wenn vor dem Erfassen der genutzten Anlagenfunktionen zunächst geprüft wird, ob die für die mindestens eine, ausgewählte Anlagenfunktion erforderlichen Hardware-Komponenten und/oder Eingangs- oder Ausgangskanäle vorhanden sind.

Optional können über die Benutzeroberfläche außerdem im Verifizierungsverfahren ermittelte Informationen, z. B. die von der Sauerstoffreduzierungsanlage genutzten Anlagenfunktionen, die im Verifizierungsdatensatz enthaltenen, vorhandenen Lizenzen und Steuerungslizenzen, die von den genutzten Anlagenfunktionen und im Lizenzdatensatz enthaltenen verwendeten Lizenzen und Steuerungslizenzen, deren jeweiligen Lizenz- bzw. Steuerungslizenztyp, deren jeweilige Lizenzanzahl bzw. Steuerungslizenzanzahl, den zugeordneten Herkunftsindikator, die ermittelten Lizenzwerte, ausgegebene Meldungen, insbesondere Fehlermeldungen und Störungsmeldungen sowie abgeschaltete Anlagenfunktionen, Zeitstände des Zeitmessers usw., von einem Benutzer abgerufen werden.

Die eingangs gestellte Erfindungsaufgabe wird auch gelöst durch ein programmierbares Steuerungsmodul für eine Sauerstoffreduzierungsanlage, wobei die Sauerstoffreduzierungsanlage zum Absenken und/oder Halten eines Sauerstoffkonzentrationsniveaus in mindestens einem Schutzbereich durch Zuführung von aus mindestens einer Inertgasquelle stammendem, insbesondere mittels mindestens eines Inertgaserzeugers erzeugtem Inertgas und zum Überwachen oder Anheben des, insbesondere zuvor abgesenkten, Sauerstoffkonzentrationsniveaus in dem Schutzbereich oder einem Überwachungsbereich ausgebildet ist, konfiguriert zur Durchführung eines Verifizierungsverfahrens nach einer der zuvor beschriebenen Varianten, mit einem oder mehreren Signaleingangskanälen und einem oder mehreren Signalausgangskanälen und mit mindestens einer Schnittstelle zur Übertragung von Daten und/oder zur Verbindung mit einem Speichermedium.

Gemäß einer vorteilhaften Ausführungsform des programmierbaren Speichermoduls ist die Anzahl an Signaleingangskanälen und/oder Signalausgangskanälen durch Hinzufügen von Hardware-Komponenten erweiterbar.

Indem also zusätzlicher Raum für die Erweiterung durch optionale Baugruppen und/oder Steckkarten vorgesehen ist, wird eine modulare und bedarfsweise Anpassung des Steuerungsmoduls an die genutzten Anlagenfunktionen und die im erfindungsgemäßen Verifizierungsverfahren genutzten, variabel zusammengestellten Lizenz- und Verifizierungsdatensätze ermöglicht.

Schließlich wird die Erfindungsaufgabe ferner gelöst durch eine Sauerstoffreduzierungsanlage mit mindestens einem programmierbaren Steuerungsmodul nach einer der oben beschriebenen Ausführungsformen und geeignet zur Durchführung eines Verifizierungsverfahren nach einer der zuvor beschriebenen Verfahrensvarianten, wobei ein oder mehrere Signaleingangskanäle des mindestens einen Steuerungsmoduls mit Sensoren der Sauerstoffreduzierungsanlage und ein oder mehrere Signalausgangskanäle mit Aktoren der Sauerstoffreduzierungsanlage verbunden sind.

Weitere Einzelheiten, Merkmale, Merkmals(unter)kombinationen, Vorteile und Wirkungen auf der Basis der Erfindung ergeben sich aus der nachfolgenden Beschreibung eines bevorzugten Ausführungsbeispiels der Erfindung und den Zeichnungen. Diese zeigen in
Fig. 1 eine schematische Darstellung einer Sauerstoffreduzierungsanlage mit einem dezentralen Steuerungs- und Regelungssystem,
Fig. 2 eine schematische Darstellung einer beispielhaften Ausführungsform zweier erfindungsgemäßer Steuerungsmodule,
Fig. 3 ein Blockdiagramm zur schematischen Veranschaulichung eines beispielhaften Ablaufs des erfindungsgemäßen Verifizierungsverfahrens,
Fig. 4 eine schematische Darstellung einer beispielhaften Ausführungsform der Erfindung, bei der mehrere programmierbare Steuerungsmodule signalleitend miteinander verbunden sind,
Fig. 5 eine schematische Darstellung einer beispielhaften Ausführungsform der in einem Steuerungsmodul implementierten Zuordnung, bei der verwendete Lizenzen vorhandenen Lizenzen zugeordnet sind,
Fig. 6 eine schematische Darstellung einer beispielhaften Ausführungsform eine globalen Lizenzdatensatzes,
Fig. 7 eine schematische Darstellung einer beispielhaften Ausführungsform der in einem Steuerungsmodul implementierten Zuordnung, bei der verwendete Steuerungslizenzen vorhandenen Steuerungslizenzen zugeordnet sind,
Fig. 8 eine erste bespielhafte Darstellung von auf einer Benutzeroberfläche angezeigten Informationen und in
Fig. 9 eine zweite bespielhafte Darstellung von auf einer Benutzeroberfläche angezeigten Informationen.

Die Figuren sind lediglich beispielhafter Natur und dienen nur dem Verständnis der Erfindung. Die gleichen Elemente sind mit denselben Bezugszeichen versehen und werden in der Regel nur einmal beschrieben.

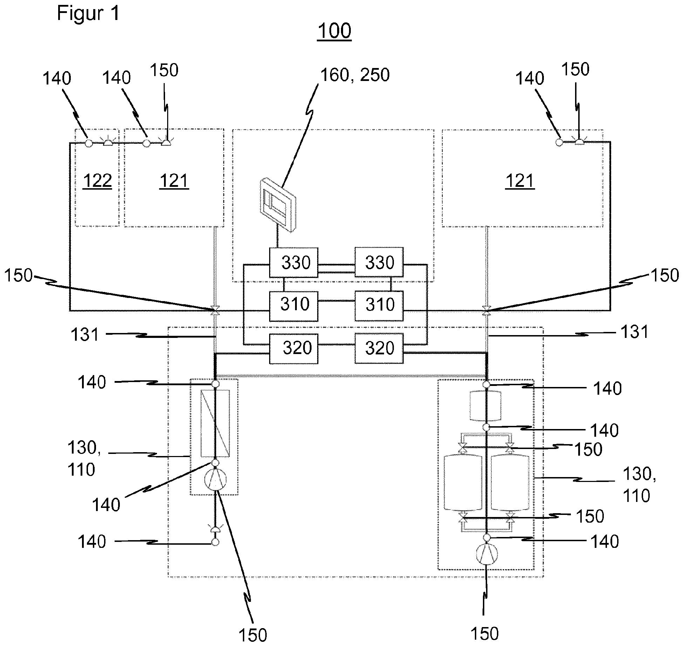

Die Figur 1 zeigt ein Ausführungsbeispiel einer erfindungsgemäßen Sauerstoffreduzierungsanlage 100, die mit einem dezentralen Steuerungs- und Regelungssystem ausgestattet ist. Die Sauerstoffreduzierungsanlage 100 umfasst zwei als Inertgaserzeuger 130 ausgebildete Inertgasquellen, die über Inertgas 131 leitende Leitungsverbindungen mit zwei Schutzbereichen 121 verbunden sind. Benachbart zu einem der Schutzbereiche 121 ist ein Überwachungsbereich 122 angeordnet. Den Inertgaserzeugern 130 ist jeweils ein zur Steuerung und Regelung des entsprechenden Inertgaserzeugers 130 konfiguriertes Prozesssteuerungsmodul 320 zugeordnet, dessen hier nicht dargestellte Signaleingangskanäle 301 an Sensoren 140, insbesondere Druck- und Sauerstoffkonzentrationssensoren und dessen hier ebenfalls nicht dargestellte Signalausgangskanäle 302 an Aktoren 150, insbesondere Ventile und Kompressoren des Inertgaserzeugers 130 angebunden sind. In entsprechender Weise ist auch den Schutzbereichen 121 jeweils ein zum Absenken und/oder Halten sowie zur Überwachung oder zum Anheben eines, gegebenenfalls zuvor abgesenkten, Sauerstoffkonzentrationsniveaus innerhalb des entsprechenden Schutzbereichs 121 konfiguriertes Bereichssteuerungsmodul 310 zugeordnet. Einem der Bereichssteuerungsmodule 310 ist außerdem ein Überwachungsbereich 122 zugeordnet. Die hier nicht dargestellten Signaleingangskanäle 301 der Bereichssteuerungsmodule 310 sind an Sensoren 140, insbesondere Sauerstoffkonzentrationssensoren, die innerhalb der jeweils zugeordneten Schutzbereiche 121 bzw. innerhalb des Überwachungsbereichs 122 angeordnet sind, angebunden. Die hier ebenfalls nicht dargestellten Signalausgangskanäle 302 sind entsprechend an Aktoren 150, insbesondere an Bereichsventile und/oder Alarmmittel, die innerhalb der jeweils zugeordneten Schutzbereiche 121 bzw. innerhalb des Überwachungsbereichs 122 angeordnet sind, angebunden.

Die Bereichssteuerungsmodule 310 und die Prozesssteuerungsmodule 320 kommunizieren nicht direkt miteinander, sondern sind mit zwei redundanten Masterreglern 330, vorzugsweise über ein Bus- oder Ringbussystem, signalleitend verbunden. Die Bereichssteuerungsmodule 310, die Prozessteuerungsmodule 320 und die Mastersteuerungsmodule 330 sind jeweils zur Durchführung des erfindungsgemäßen Verifizierungsverfahrens 200 konfiguriert, wobei insbesondere auf den Bereichsreglern 310 und den Prozessreglern 320 ein Erfassen 201 der dort lokal genutzten Anlagenfunktionen 110 und auf den Masterreglern 330 ein Einlesen eines Verifizierungsdatensatzes 220 implementiert ist. Über ein mit einem der Mastersteuerungsmodule 330 signalverbundenes Bedien- und Anzeigepanel 160 kann außerdem eine Benutzeroberfläche 250, zur Darstellung der im Verifizierungsverfahren 200 ermittelten Informationen angezeigt werden.

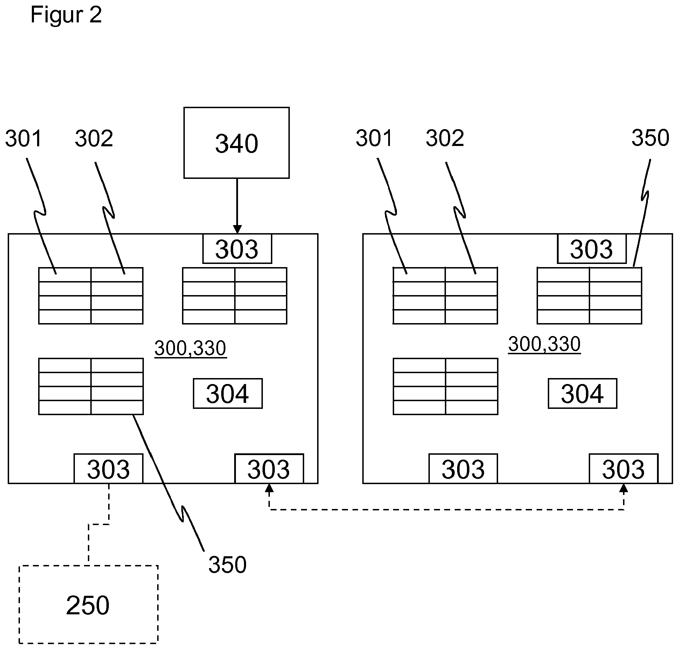

In der Figur 2 ist eine schematische Darstellung einer beispielhaften Ausführungsform zweier erfindungsgemäßer Steuerungsmodule 300 dargestellt. Die beiden Steuerungsmodule 300 sind vorzugsweise jeweils als Mastersteuerungsmodule 330 konfiguriert, weisen einen identischen Aufbau auf und bilden vorzugsweise zur Erhöhung der Ausfallsicherheit eine Redundanz. Ein Steuerungsmodul 300 umfasst hier beispielhaft drei Hardware-Komponenten 350, die jeweils insbesondere vier Signaleingangskanäle 301 zur Anbindung an Sensoren 140 der Sauerstoffreduzierungsanlage 100 sowie vier Signalausgangskanäle 302 zur Anbindung an Aktoren 150 der Sauerstoffreduzierungsanlage 100 aufweisen. Die Anzahl der Hardware-Komponenten 350 kann bedarfsweise modular erweitert werden. Anhand der Belegung der Signaleingangskanäle 301 und/oder der Signalausgangskanäle 302 können die von der Sauerstoffreduzierungsanlage 100 genutzten Anlagenfunktionen 110 erkannt und erfasst werden. Das Steuerungsmodul 300 weist ferner einen Zeitmesser 304 und z. B. drei Schnittstellen 303 auf, wobei eine der Schnittstellen 303 mit einem externen Speichermedium 340 signalverbunden ist. Eine zweite Schnittstelle 303 kann zur signalleitenden Verbindung mit einem Eingabe- und Ausgabegerät, bspw. einem Computer oder Laptop, mit entsprechender Benutzeroberfläche 250 vorgesehen sein. Schließlich ist eine dritte Schnittstelle 303 zur signalleitenden Verbindung mit einem Bus-, insbesondere Ringbussystem oder einem sonstigen Netzwerk vorgesehen. In der hier gezeigten Abbildung ist beispielhaft eine signalleitende Verbindung der Schnittstelle 303 mit dem redundanten Steuerungsmodul 300 dargestellt.

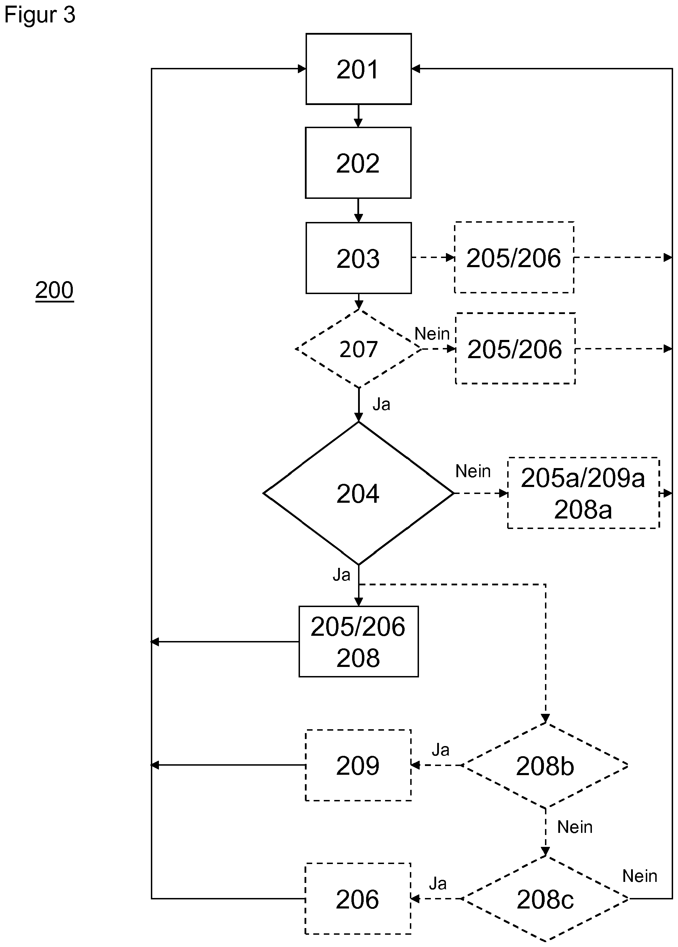

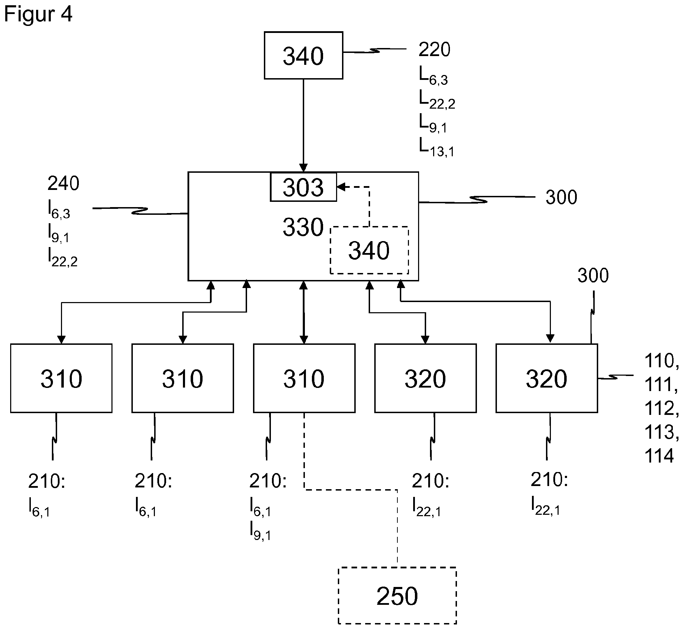

Ein Blockdiagramm zur schematischen Veranschaulichung eines beispielhaften Verfahrensablaufs des erfindungsgemäßen Verifizierungsverfahrens 200 kann der Figur 3 entnommen werden und wird nachfolgend auch unter Bezugnahme auf die Figur 4, die eine beispielhafte Anordnung mehrerer Steuerungsmodule 300 für eine Sauerstoffreduzierungsanlage 100 darstellt, näher erläutert. Das Verifizierungsverfahren 200 wird vorzugsweise im Betrieb der Sauerstoffreduzierungsanlage 100 durchgeführt, d. h. während eine oder mehrere Anlagenfunktionen 110 von der Sauerstoffreduzierungsanlage 100 genutzt werden. Gemäß der Figur 4 ist das Verifizierungsverfahren 200 auf z. B. sechs untereinander signalverbundenen, programmierbaren Steuerungsmodulen 300 implementiert, wobei eines der Steuerungsmodule 300 als Mastersteuerungsmodul 330, drei weitere Steuerungsmodule 300 als Bereichssteuerungsmodule 310 und die verbleibenden zwei Steuerungsmodule 300 als Prozesssteuerungsmodule 320 konfiguriert sind. Die Bereichssteuerungsmodule 310 sind dabei zur Steuerung und/oder Regelung von lokal in einem zugeordneten Schutz- oder Überwachungsbereich 121, 122 durchzuführenden Anlagenfunktionen 110 konfiguriert und die Prozesssteuerungsmodule 320 entsprechend zur Steuerung und/oder Regelung der Anlagenfunktionen 110 jeweils zugeordneter Inertgasquellen, hier im Beispiel Inertgaserzeuger 130. Auch das Mastersteuerungsmodul 330 kann zur Steuerung und/oder Regelung lokal genutzter Anlagenfunktionen 110 konfiguriert sein.

Zurück zur Figur 3 werden in einem ersten Verfahrensschritt 201 die von der Sauerstoffreduzierungsanlage 100 genutzten Anlagenfunktionen 110 erfasst. Die Erfassung 201 der Anlagenfunktionen 100 ist zweckmäßig auf jedem der Steuerungsmodule 300, 310, 320, 330 implementiert, sodass dort jeweils genutzte Anlagenfunktionen 110 lokal, anhand der individuellen Eingangs- und Ausgangskanalbelegung des jeweiligen Steuerungsmoduls 300, 310, 320, 330 erfasst werden.

In einem zweiten Verfahrensschritt 202 wird anhand der erfassten Anlagenfunktionen 110 ein Lizenzdatensatz 210 erstellt. Vorzugsweise ist auch der zweite Verfahrensschritt 202 lokal auf jedem Steuerungsmodul 300, 310, 320, 330 implementiert, sodass auf jedem Steuerungsmodul 300, 310, 320, 330 ein individueller Lizenzdatensatz 210 lokal erstellt werden kann. Hierzu werden die Anlagenfunktionen 110 zunächst in lizenzpflichtige Anlagenfunktionen 111 und lizenzfreie Anlagenfunktionen 112 unterschieden. Grundsätzlich wird für jede lizenzpflichtige Anlagenfunktion 111 eine verwendete Lizenz l_{ny}, deren Lizenztyp n der Anlagenfunktion 111 entspricht (z.B. n = 9: Luftumwälzung) und deren erste Lizenzanzahl y der Anzahl der für die Anlagenfunktion 111 verwendeten Lizenzen entspricht (z.B. y = 1), im Lizenzdatensatz 210 hinterlegt. Bei der Luftumwälzung handelt es sich ferner um eine sogenannte optionale Anlagenfunktion 114, die anders als die sogenannten Basisanlagenfunktionen 113, für den Betrieb der Sauerstoffreduzierungsanlage 100 nicht zwingend erforderlich ist. Zweckmäßigerweise kann für mehrere Basisanlagenfunktionen 113 eine einzige verwendete Lizenz l_{ny}, eine Basislizenz, deren Lizenztyp n den Basisanlagenfunktionen 113 entspricht (z.B. n = 6: Bereichssteuerungsmodul (Standardfunktionen) oder n = 22: Prozesssteuerungsmodul (Standardfunktionen)) im Lizenzdatensatz 210 hinterlegt werden.

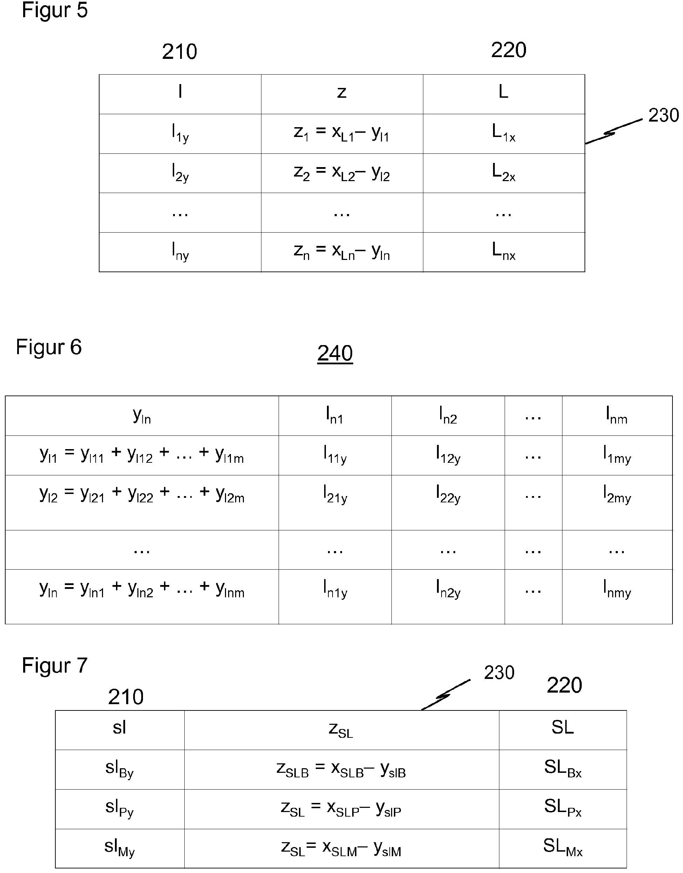

Im vorliegenden Ausführungsbeispiel gemäß der Figur 4, sofern also mehr als ein Lizenzdatensatz 210 lokal erstellt wird, werden die lokal erstellten Lizenzdatensätze 210 zu einem einzigen, globalen Lizenzdatensatz 240 zusammengefasst (siehe auch Figur 6). Dieser Verfahrensschritt ist zweckmäßigerweise auf dem Mastersteuerungsmodul 330 implementiert. Der globale Lizenzdatensatz 240 umfasst die jeweilige Summe, der insgesamt verwendeten Lizenzen (l_{1y}, l_{2y} ..., l_{ny}) des jeweiligen Lizenztyps n sowie die dem entsprechenden Steuerungsmodul 310, 320, 330 zugeordnete, lokale Lizenzanzahl y. Hierzu werden die jeweiligen verwendeten Lizenzen (l_{1my}, l_{2my} ..., l_{nmy}) mit einem zusätzlichen Herkunftsindikator m versehen (siehe auch Figur 6). Der globale Lizenzdatensatz 240 enthält demnach eine verwendete Lizenz l_{6,3} vom Lizenztyp Bereichssteuerungsmodul (Standardfunktionen) und mit der ersten Lizenzanzahl y = 3, eine verwendete Lizenz l_{22,2} vom Lizenztyp Prozesssteuerungsmodul (Standardfunktionen) und mit der ersten Lizenzanzahl y = 2 sowie eine verwendete Lizenz l_{9,1} vom Lizenztyp Luftumwälzung und mit der ersten Lizenzanzahl y =1 .Zusätzlich sind Information darüber enthalten, welches Steuerungsmodul 300, 310, 320, 330 die jeweiligen Lizenzen verwendet.

Nach den Figuren 3 und 4 wird in einem dritten Verfahrensschritt 203 ein auf einem Speichermedium 340 bereitgestellter Verifizierungsdatensatz 220, der eine Anzahl an vorhandenen Lizenzen (L₁ₓ, L₂ₓ ... Lₙₓ) enthält eingelesen und in einem vierten Verfahrensschritt 204 eine Lizenzverletzung festgestellt, sofern nicht jede verwendete Lizenz (l_{1y}, l_{2y}, ..., l_{ny}) des Lizenzdatensatzes 210 (Lizenztyp n und jeweilige Lizenzanzahl y) oder gemäß dem vorliegenden Ausführungsbeispiel, des globalen Lizenzdatensatzes 240 mit den verwendeten Lizenzen l_{6,3}, l_{22,2}, l_{9,1} von dem Verifizierungsdatensatz 220 umfasst ist. So würde zum Beispiel bei einem Verifizierungsdatensatz 220 der nur die vorhandenen Lizenzen L_{6,3}, L_{22,2} oder nur die vorhandenen Lizenzen L_{6,2}, L_{22,2}, L_{9,1} enthält jeweils eine Lizenzverletzung festgestellt.

Je nach Verfahrensvariante kann bei Vorliegen einer Lizenzverletzung in einem fünften Verfahrensschritt 205, 206 wahlweise entweder eine Meldung, insbesondere eine Fehlermeldung ausgegeben werden 205 oder die Lizenzverletzung hat ein Abschalten 206 mindestens einer, insbesondere einer optionalen und/oder lizenzpflichtigen Anlagenfunktion 111, 114 oder alternativ aller Anlagenfunktionen 110, 111, 112, 113, 114 zur Folge. Sofern gemäß dem Ausführungsbeispiel der Verifizierungsdatensatz 220 mindestens die vorhandenen Lizenzen L_{6,3}, L_{22,2}, L_{9,1}, enthält, wird eine Lizenzverletzung nicht festgestellt und das Verifizierungsverfahren 200 wird beginnend mit dem ersten Verfahrensschritt 201 wiederholt. Zweckmäßigerweise kann der Verifizierungsdatensatz 220 auch weitere vorhandene Lizenzen L enthalten, wie hier gezeigt beispielsweise eine vorhandene Lizenz L_{13,1} vom Lizenztyp n = 13: Überwachung Zugangstüren mit der zweiten Lizenzanzahl x = 1, die einem Benutzer oder dem Service-Personal erlauben, zusätzliche Anlagenfunktionen 110 zu nutzen, die z. B. im vorgesehenen Lizenzumfang enthalten sind und deren Nutzung daher nicht zur Feststellung einer Lizenzverletzung führen soll.

Auch nachdem eine Meldung, insbesondere eine Fehlermeldung oder eine Störmeldung, ausgegeben wurde 205, wird das erfindungsgemäße Verifizierungsverfahren 200 wiederholt durchgeführt. Unabhängig von der Feststellung einer Lizenzverletzung wird der Betrieb der Sauerstoffreduzierungsanlage 100 (zunächst) fortgesetzt. Vorzugsweise wird das erfindungsgemäße Verifizierungsverfahren 200 in regelmäßigen zeitlichen Abständen wiederholt, wobei lediglich ein Abschalten 206 aller Anlagenfunktionen 110, 111, 112, 113, 114 auch zu einer Beendigung des erfindungsgemäßen Verifizierungsverfahrens 200 führt. Um den Betrieb der Sauerstoffreduzierungsanlage 100 wieder aufzunehmen und das Verifizierungsverfahren 200 weiter zu durchzuführen, ist ein manueller Neustart der Sauerstoffreduzierungsanlage 100 und gegebenenfalls auch des Verifizierungsverfahrens 200 erforderlich.

Gemäß einer Verfahrensvariante kann in einem optionalen Verfahrensschritt 208 mit Feststellung 204 einer Lizenzverletzung, ein Zeitmesser 304 gestartet werden, welcher das verstrichene Zeitintervall zwischen der ersten Feststellung 204 einer Lizenzverletzung, bis in einem nachfolgenden Verfahrensdurchlauf eine Prüfung auf Lizenzverletzung erstmals negativ ausfällt, d. h. die Lizenzverletzung nicht länger vorliegt, misst. Nach einer weiteren Verfahrensvariante kann nach Ablauf, d. h. sofern der Zeitmesser 304 ausgehend von einem ersten vorgegebenen Zeitintervall (z. B. 60 Minuten) rückwärts misst oder mit Erreichen des ersten vorgegebenen Zeitintervalls, d. h. sofern der Zeitmesser ausgehend von einem Zeitwert Null vorwärts misst, in welchem Zeitintervall eine Fehlermeldung ununterbrochen ausgegeben wird 205, eine Störmeldung ausgegeben werden 209. Nach Ablauf oder mit Erreichen eines zweiten Zeitintervalls (z. B. 72 Stunden) in welchem die Störmeldung ununterbrochen ausgegeben wird, können dann vorzugsweise alle Anlagenfunktionen abgeschaltet 206 und das Verifizierungsverfahren 200 beendet werden.

Wird zu einem Zeitpunkt des Verifizierungsverfahrens 200 festgestellt dass eine Lizenzverletzung nicht vorliegt und wenn eine Fehlermeldung und/oder Störmeldung ausgegeben wird 205, 209, kann in einem optionalen Verfahrensschritt 205a, 209a die Fehlermeldung und/oder Störmeldung zurückgesetzt werden, bevor ein nachfolgender Durchlauf des Verifizierungsverfahrens 200 gestartet wird. Entsprechend kann der Zeitmesser 304 in einem optionalen Verfahrensschritt 208a zurückgesetzt werden, sofern zu einem Zeitpunkt des Verifizierungsverfahrens 200 festgestellt wird 204, dass eine Lizenzverletzung nicht vorliegt und der Zeitmesser 304 zur Erfassung eines Zeitintervalls gestartet wurde bzw. eine Zeitwert ungleich Null ausgibt.

In einem optionalen Verfahrensschritt 207 kann außerdem zur Erhöhung der Sicherheit geprüft werden, ob der Verifizierungsdatensatz 220 einen Freischaltcode enthält, wobei eine Meldung, insbesondere eine Störmeldung, ausgegeben wird 209 und/oder mindestens eine Anlagenfunktion 110 abgeschaltet wird 206, sofern der Freischaltcode nicht erkannt wird. Der Verfahrensschritt 207 wird vorzugsweise nach dem dritten Verfahrensschritt 203 durchgeführt, kann aber auch davor oder zeitgleich erfolgen.

Das Einlesen 203 des Verifizierungsdatensatzes 210 erfolgt vorzugsweise über eine mit dem Speichermedium 340 signalübertragend verbundene oder verbindbare Schnittstelle 303. Schließlich kann optional außerdem vorgesehen sein, eine Meldung, insbesondere eine Störmeldung, auszugeben 209 und/oder mindestens eine Anlagenfunktion 110 abzuschalten, sofern die signalübertragende Verbindung zwischen dem Speichermedium 340 und der Schnittstelle 303 nicht erkannt wird.

In der Figur 5 ist eine matrixartige, d.h. spalten- und reihenweise Zuordnung 230 der im Lizenzdatensatz 210 enthaltenen, verwendeten Lizenzen (l_{1y}, l_{2y} ..., l_{ny}) zu den im Verifizierungsdatensatz 220 enthaltenen, vorhandenen Lizenzen (L₁ₓ, L₂ₓ ... Lₙₓ) dargestellt. Die verwendeten Lizenzen (l_{1y}, l_{2y} ..., l_{ny}) sind hier beispielhaft in Spalte eins aufgelistet, die vorhandenen Lizenzen (L₁ₓ, L₂ₓ ... Lₙₓ) in Spalte drei. Jede Reihe entspricht dabei einem bestimmten Lizenztyp n, sodass Lizenzen desselben Lizenztyps n reihenweise einander zugeordnet sind. In der zweiten bzw. mittleren Spalte ist der aus der Differenz der jeweiligen Lizenzanzahlen x_{Ln}- yₗₙ reihenweise gebildete Lizenzwert z aufgelistet. Sofern einer der gebildeten Lizenzwerte z < 0 ist, wird eine Lizenzverletzung festgestellt.

Eine schematische Darstellung eines beispielhaften globalen Lizenzdatensatzes 240 ist in der Figur 6 gezeigt. In der ersten Spalte wird die, einem jeweiligen Lizenztyp n zugeordnete Summe aller Lizenzanzahlen yₗₙₘ gebildet. Die weiteren Spalten enthalten, aufgeschlüsselt nach jeweils unterschiedlichen Steuerungsmodulen 300 die dort lokal verwendeten Lizenzen l_{nmy}, wobei in dem globalen Lizenzdatensatz 240 jeder verwendeten Lizenz l_{nmy} zusätzlich ein Herkunftsindikator m zugeordnet ist, sodass von demselben Steuerungsmodul 300 verwendete Lizenzen l_{nmy} einander spaltenweise und Lizenzen l_{nmy} desselben Lizenztyps n einander reiheinweise zugeordnet sind.

Figur 7 stellt entsprechend der Figur 5 eine tabellenartige und/oder matrixartige, d.h. spalten- und reihenweise Zuordnung 230 der im Lizenzdatensatz 210 enthaltenen, verwendeten Steuerungsmodullizenzen (sl_{By}, sl_{Py}, sl_{My}) zu den im Verifizierungsdatensatz 220 enthaltenen, vorhandenen Steuerungsmodullizenzen (SL_{BX}, SL_{PX}, SL_{MX}) dar. Anstelle des Lizenztyps n sind die verwendeten Steuerungsmodullizenzen (sl_{By}, sl_{Py}, sl_{My}) und die vorhandenen Steuerungsmodullizenzen (SL_{BX}, SL_{PX}, SL_{MX}) hier jeweils mit einem Parameter zur Kennzeichnung des Steuerungsmodultyps versehen, wobei "B" ein Bereichssteuerungsmodul 310, "P" ein Prozesssteuerungsmodul 320 und "M" ein Mastersteuerungsmodul 330 kennzeichnet. In der mittleren Spalte werden drei Steuerungsmodullizenzwerte z_{SL} gebildet die die Differenz zwischen den verwendeten Steuerungsmodullizenzen (sl_{By}, sl_{Py}, sl_{My}) und den vorhandenen Steuerungsmodullizenzen (SL_{BX}, SL_{PX}, SL_{MX}) wiedergeben. Eine Lizenzverletzung wird festgestellt, sofern einer der gebildeten Steuerungsmodullizenzwerte z_{SL} < 0 ist.

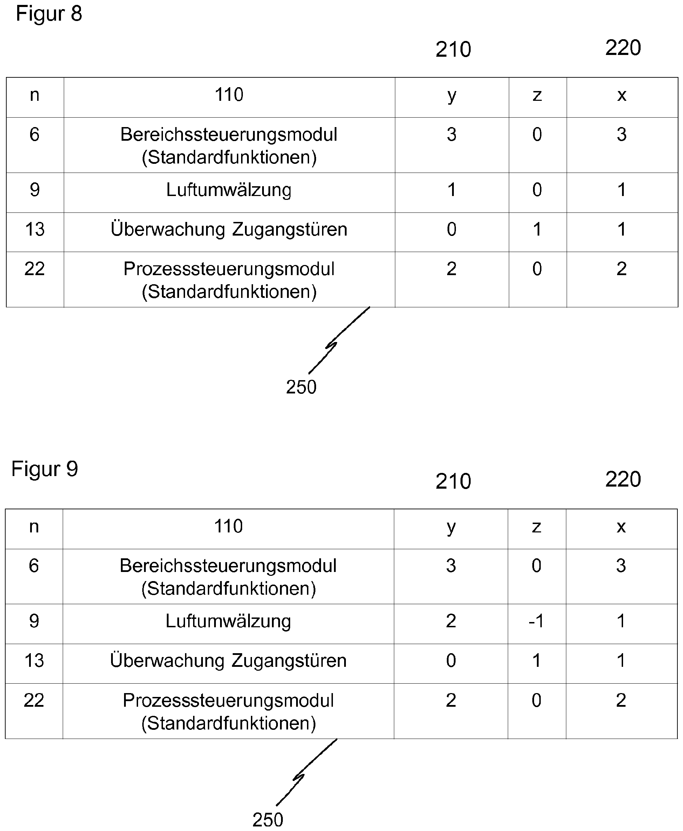

In den Figuren 8 und 9 wird eine spalten- und reihenweise Zuordnung 230, wie sie in der Figur 5 in allgemeiner Form dargestellt ist, anhand des bereits zuvor verwendeten Ausführungsbeispiels gemäß Figur 3 veranschaulicht. In der ersten Spalte der Tabelle sind für das Verifizierungsverfahren 200 hinterlegte Lizenztypen n = 6, 9, 13, 22 aufgelistet. In Spalte zwei ist veranschaulicht, wie für einen Benutzer oder das Bedienpersonal der Sauerstoffreduzierungsanlage 100 anhand einer Benutzeroberfläche 250 die Realnamen der den jeweiligen Lizenztypen n zugeordneten Anlagenfunktionen 110 angezeigt werden können: n = 6: Bereichssteuerungsmodul (Standardfunktionen), n = 9: Luftumwälzung, n = 13: Überwachung Zugangstüren und n = 22: Prozesssteuerungsmodul (Standardfunktionen). In der Spalte drei ist dann die jeweilige erste Lizenzanzahl y der verwendeten Lizenzen l hinterlegt und kann dem Benutzer als konkreter Wert angezeigt werden. Die jeweilige zweite Lizenzanzahl x der vorhandenen Lizenzen L ist in der Spalte fünf hinterlegt und wird für einen Benutzer oder das Servicepersonal auf der Benutzeroberfläche 250 ebenfalls als konkreter Wert ausgegeben. Die aus der ersten Lizenzanzahl y der verwendeten Lizenzen l und der zweiten Lizenzanzahl x der vorhandenen Lizenzen L gebildete Differenz ist als Lizenzwert z hinterlegt und informiert den Benutzer oder das Servicepersonal auf der Benutzeroberfläche 250 über die Anzahl an derzeit verfügbaren Lizenzen. Gemäß dem Ausführungsbeispiel werden zum derzeitigen Zeitpunkt alle auf dem Verifizierungsdatensatz 220 vorhandenen Lizenzen L, mit Ausnahme einer Lizenz zur Überwachung der Zugangstüren verwendet. Die Lizenz zur Überwachung der Zugangstüren ist frei verfügbar und könnte zur Durchführung einer entsprechenden Anlagenfunktion 110 genutzt werden, indem der Benutzer die entsprechende, auf einem Steuerungsmodul 300 hinterlegte Anlagenfunktion 110 manuell auswählt.

Die in der Figur 9 dargestellte Tabelle enthält im Wesentlichen identische Informationen, stellt jedoch einen späteren Zeitpunkt während des Betriebs der Sauerstoffreduzierungsanlage 100 dar. Zusätzlich zu den bislang genutzten Anlagenfunktionen 110 wurde von einem Benutzer oder dem Service-Personal die Anlagenfunktion 110 Luftumwälzung an einem Steuerungsmodul 300 ausgewählt. In Spalte 3 hat sich der Wert der ersten Lizenzanzahl y, der die Anzahl der verwendeten Lizenzen Luftumwälzung wiedergibt entsprechend um 1 erhöht, es werden zwei Lizenzen Luftumwälzung verwendet. Gemäß dem Ausführungsbeispiel liegt auch zu diesem Zeitpunkt derselbe Verifizierungsdatensatz 220 vor, weshalb sich bezüglich der Anlagenfunktion 110 Luftumwälzung ein entsprechender Lizenzwert von -1 ergibt. Im Verifizierungsverfahren 200 wird eine Lizenzverletzung festgestellt 204 und entsprechend eine Meldung ausgegeben 205. Anhand der auf der Benutzeroberfläche 250 dargestellten Informationen kann der Benutzer erkennen, aufgrund von welcher Anlagefunktion 110 die Lizenzverletzung verursacht wurde und gegebenenfalls beheben.

### Bezugszeichenliste

- 100: Sauerstoffreduzierungsanlage
- 110: Anlagenfunktionen
- 111: lizenzpflichtige Anlagenfunktionen
- 112: lizenzfreie Anlagenfunktionen
- 113: Basisanlagenfunktionen
- 114: optionale Anlagenfunktionen
- 121: Schutzbereich
- 122: Überwachungsbereich
- 130: Inertgasquelle, insbesondere Inertgaserzeuger
- 131: Inertgas
- 140: Sensor
- 150: Aktor
- 160: Bedien- und Anzeigepanel
- 200: Verifizierungsverfahren
- 201: Erfassen von Anlagenfunktionen, erster Verfahrensschritt
- 202: Erstellen eines Lizenzdatensatzes, zweiter Verfahrensschritt
- 203: Einlesen eines Verifizierungsdatensatzes, dritter Verfahrensschritt
- 204: Feststellen einer Lizenzverletzung, vierter Verfahrensschritt
- 205: Ausgeben einer Meldung, insbesondere Fehlermeldung, fünfter Verfahrensschritt
- 205a: Zurücksetzen einer Fehlermeldung
- 206: Abschalten mindestens einer Anlagenfunktion, fünfter Verfahrensschritt
- 207: Prüfen auf Freischaltcode
- 208: Starten eines Zeitmessers
- 208a: Zurücksetzen eines Zeitmessers
- 208b: Ablauf oder Erreichen eines ersten Zeitintervalls
- 208c: Ablauf oder Erreichen eines zweiten Zeitintervalls
- 209: Ausgeben einer Störmeldung
- 209a: Zurücksetzen einer Störmeldung
- 210: Lizenzdatensatz
- 220: Verifizierungsdatensatz
- 230: Zuordnung
- 240: globaler Lizenzdatensatz
- 250: Benutzeroberfläche
- 300: programmierbares Steuerungsmodul
- 301: Signaleingangskanal
- 302: Signalausgangskanal
- 303: Schnittstelle
- 304: Zeitmesser
- 310: Bereichssteuerungsmodul
- 320: Prozesssteuerungsmodul
- 330: Mastersteuerungsmodul
- 340: Speichermedium
- 350: Hardware-Komponente

- I: verwendete Lizenzen (l_{1y}, l_{2y} ..., l_{ny})
- L: vorhandene Lizenzen (L₁ₓ, L₂ₓ ... Lₙₓ)
- sl: verwendete Steuerungsmodullizenzen (sl_{By}, sl_{Py}, sl_{My})
- SL: vorhandene Steuerungsmodullizenzen (SL_{BX}, SL_{PX}, SL_{MX})
- n: Lizenztyp
- m: Herkunftsindikator
- x: zweite Lizenzanzahl (x = 0, 1, ..., n)
- y: erste Lizenzanzahl (y = 0, 1, ..., n)
- z: Lizenzwert (z = -n, ..., -1, 0, 1, ..., n)
- x_{SL}: zweite Steuerungsmodullizenzanzahl (x = 0, 1, ..., n)
- yₛₗ: erste Steuerungsmodullizenzanzahl (y = 0, 1, ..., n)
- z_{SL}: Steuerungsmodullizenzwert (z_{SL1}, z_{SL2}, ..., z_{SLn})

## Patentansprüche

1. Verifizierungsverfahren (200) zur Verifizierung von im Betrieb einer Sauerstoffreduzierungsanlage (100) genutzten Anlagenfunktionen (110, 111, 112, 113, 114) der Sauerstoffreduzierungsanlage (100), wobei die Sauerstoffreduzierungsanlage (100) zum Absenken und/oder Halten eines Sauerstoffkonzentrationsniveaus in mindestens einem Schutzbereich (121) durch Zuführung von aus mindestens einer Inertgasquelle (130) stammendem Inertgas (131) und zum Überwachen oder Anheben des, insbesondere zuvor abgesenkten, Sauerstoffkonzentrationsniveaus in dem Schutzbereich (121) oder einem Überwachungsbereich (122) ausgebildet ist, die nachfolgenden Verfahrensschritte aufweisend:
- Erfassen (201) von im Betrieb der Sauerstoffreduzierungsanlage (100) genutzten Anlagenfunktionen (110, 111, 112, 113, 114),
- Erstellen (202) eines Lizenzdatensatzes (210), wobei der Lizenzdatensatz (210) von den genutzten Anlagenfunktionen (110, 111, 112, 113, 114) verwendete Lizenzen (l_{1y}, l_{2y} ..., l_{ny}) enthält,
- Einlesen (203) eines auf einem Speichermedium (340) bereitgestellten Verifizierungsdatensatzes (220), wobei der Verifizierungsdatensatz (220) mindestens eine vorhandene Lizenz (L₁ₓ, L₂ₓ ... Lₙₓ) enthält,
- Feststellen (204) einer Lizenzverletzung, sofern nicht jede verwendete Lizenz (l_{1y}, l_{2y}, ..., l_{ny}) von dem Verifizierungsdatensatz (220) umfasst ist,
- Ausgeben (205) einer Meldung, insbesondere einer Fehlermeldung und/oder Abschalten (206) mindestens einer Anlagenfunktion (110, 111, 112, 113, 114), sofern eine Lizenzverletzung festgestellt wird, und wobei das Verifizierungsverfahren (200) auf mindestens einem programmierbaren Steuerungsmodul (300, 310, 320, 330) implementiert ist und die genutzten Anlagenfunktionen (110, 111, 112, 113, 114) anhand einer spezifischen Belegung von Signaleingangs- und Signalausgangskanälen (301, 302) des mindestens einen programmierbaren Steuerungsmoduls (300, 310, 320, 330) erfasst werden.

2. Verifizierungsverfahren (200) nach Anspruch 1,
**dadurch gekennzeichnet, dass**
jede verwendete Lizenz (l_{1y}, l_{2y}, ..., l_{ny}) des Lizenzdatensatzes (210) mindestens zwei Parameter, einen jeweiligen Lizenztyp (1, 2, ..., n) und eine dem jeweiligen Lizenztyp (1, 2, ..., n) zugeordnete erste Lizenzanzahl (yₗ₁, yₗ₂, ... yₗₙ) und jede vorhandene Lizenz (L₁ₓ, L₂ₓ ... Lₙₓ) des Verifizierungsdatensatzes (220) mindestens zwei Parameter, einen jeweiligen Lizenztyp (1, 2, ..., n) und eine dem jeweiligen Lizenztyp (1, 2, ..., n) zugeordnete zweite Lizenzanzahl (x_{L1}, x_{L2}, ... x_{Ln}) umfasst und zum Feststellen (204) einer Lizenzverletzung eine, insbesondere matrixartige, Zuordnung (230) der verwendeten Lizenzen (l_{1y}, l_{2y}, ..., l_{ny}) zu den vorhandenen Lizenzen (L₁ₓ, L₂ₓ ... Lₙₓ) erfolgt.

3. Verifizierungsverfahren (200) nach Anspruch 2,
**dadurch gekennzeichnet, dass**
zum Feststellen (204) einer Lizenzverletzung mindestens ein Lizenzwert (z₁, z₂, ..., zₙ) jeweils einander zugeordneter Lizenztypen (1, 2, ..., n) ermittelt wird, indem die erste Lizenzanzahl (yₗ₁, yₗ₂, ... yₗₙ) der verwendeten Lizenzen (l_{1y}, l_{2y}, ..., l_{ny}) von der zweiten Lizenzanzahl (x_{L1}, x_{L2}, ... x_{Ln}) der vorhandenen Lizenzen (L₁ₓ, L₂ₓ ... Lₙₓ) subtrahiert wird, und wobei eine Lizenzverletzung festgestellt wird, sofern einer der ermittelten Lizenzwerte (z₁, z₂, ..., zₙ) < 0 ist.

4. Verifizierungsverfahren (200) nach einem der vorhergehenden Ansprüche,
**gekennzeichnet, durch**
Prüfen (207), ob der Verifizierungsdatensatz (220) einen Freischaltcode enthält, wobei eine Meldung, insbesondere eine Störmeldung, ausgegeben (205) wird und/oder mindestens eine Anlagenfunktion (110, 111, 112, 113, 114) abgeschaltet (206) wird, sofern der Freischaltcode nicht erkannt wird.

5. Verifizierungsverfahren (200) nach einem der vorhergehenden Ansprüche,
**dadurch gekennzeichnet, dass**
das Einlesen (203) des Verifizierungsdatensatzes über eine mit dem Speichermedium (340) signalübertragend verbundene oder verbindbare Schnittstelle (303) erfolgt, wobei eine Meldung, insbesondere eine Störmeldung, ausgegeben (205) wird und/oder mindestens eine Anlagenfunktion (110, 111, 112, 113, 114) abgeschaltet (206) wird, sofern die signalübertragende Verbindung zwischen dem Speichermedium (340) und der Schnittstelle (303) nicht erkannt wird.

6. Verifizierungsverfahren (200) nach einem der vorhergehenden Ansprüche,
**dadurch gekennzeichnet, dass**
das Verifizierungsverfahren (200) in bestimmten oder regelmäßigen zeitlichen Abständen wiederholt wird, und gleichzeitig bei Ausgabe der Meldung (205), insbesondere der Fehlermeldung, ein Zeitmesser (304) gestartet (208) wird, um die Zeitspanne während der die Meldung, insbesondere die Fehlermeldung, ausgegeben (205) wird zu erfassen.

7. Verifizierungsverfahren (200) nach Anspruch 6,
**dadurch gekennzeichnet, dass**
mit Erreichen oder nach Ablauf einer vorgegebenen Zeitspanne, während der die Meldung, insbesondere die Fehlermeldung, ausgegeben (205) wird, eine Störmeldung ausgegeben (209) wird und/oder mindestens eine Anlagenfunktion (110, 111, 112, 113, 114) der Sauerstoffreduzierungsanlage (100) abgeschaltet (206) wird.

8. Verifizierungsverfahren (200) nach einem der Ansprüche 6 oder 7,
**dadurch gekennzeichnet, dass**
mit Erreichen oder nach Ablauf einer vorgegebenen Zeitspanne, während der die Meldung, insbesondere die Störmeldung, ausgegeben (205) wird ein Abschalten (206) mindestens einer Anlagenfunktion (110, 111, 112, 113, 114) der Sauerstoffreduzierungsanlage (100) erfolgt.

9. Verifizierungsverfahren (200) nach einem der vorhergehenden Ansprüche,
**dadurch gekennzeichnet, dass**
das Verifizierungsverfahren (200) auf mehreren programmierbaren und miteinander signalverbundenen Steuerungsmodulen (300, 310, 320, 330) implementiert ist, wobei mindestens ein Steuerungsmodul (300) als Bereichssteuerungsmodul (310), das mindestens einem Schutzbereich (121) oder Überwachungsbereich (122) zugeordnet ist und/oder mindestens ein Steuerungsmodul (300) als Prozesssteuerungsmodul (320), das mindestens einer Inertgasquelle (130) zugeordnet ist und/oder mindestens ein Steuerungsmodul (300) als Mastersteuerungsmodul (330) vorgesehen ist.

10. Verifizierungsverfahren (200) nach Anspruch 9,
**dadurch gekennzeichnet, dass**
das Erfassen (201) genutzter Anlagenfunktionen (110, 111, 112, 113, 114) auf dem mindestens einen Bereichssteuerungsmodul (310) und/oder dem mindestens einen Prozesssteuerungsmodul (320) und/oder dem mindestens einen Mastersteuerungsmodul (330) implementiert ist, wobei dem jeweiligen Steuerungsmodul (310, 320, 330) lokal zugeordnete Anlagenfunktionen (110, 111, 112, 113, 114) anhand einer spezifischen Belegung von Signaleingangs- und Signalausgangskanälen (301, 302) des jeweiligen Bereichssteuerungsmoduls (310) und/oder Prozesssteuerungsmoduls (320) und/oder Mastersteuerungsmoduls (330) erfasst werden.

11. Verifizierungsverfahren (200) nach einem der Ansprüche 9 oder 10,
**gekennzeichnet, durch**
lokales Erstellen (202) mindestens eines Lizenzdatensatzes (210), der von dem jeweiligen Steuerungsmodul (310, 320, 330) verwendete Lizenzen (l_{1y}, l_{2y} ..., l_{ny}) enthält, wobei jede dort verwendete Lizenz (l_{1y}, l_{2y} ..., l_{ny}) mindestens einer lokal genutzten Anlagenfunktion (110, 111, 112, 113, 114) zugeordnet ist und mindestens zwei Parameter, einen jeweiligen Lizenztyp (1, 2, ..., n) und eine dem jeweiligen Lizenztyp (1, 2, ..., n) zugeordnete erste Lizenzanzahl (yn, yₗ₂, ... yₗₙ) umfasst.

12. Verifizierungsverfahren (200) nach Anspruch 11,
**dadurch gekennzeichnet, dass**
ein Erstellen (202) eines globalen Lizenzdatensatzes (240) auf dem mindestens einen Mastersteuerungsmodul (330) implementiert ist, und der globale Lizenzdatensatz (240) durch Zusammenfügen der lokal erstellten Lizenzdatensätze (210) gebildet wird, indem die ersten Lizenzanzahlen (yₗ₁, yₗ₂, ... yₗₙ) der von den jeweiligen Steuerungsmodulen (310, 320, 330) lokal verwendeten Lizenzen (l_{1y}, l_{2y}, ..., l_{ny}) jeweils einander zugeordneter Lizenztypen (1, 2, ..., n) addiert werden und wobei den von den jeweiligen Steuerungsmodulen (310, 320, 330) lokal verwendeten Lizenzen (l_{11y}, l_{22y} ..., l_{nmy}) ein dem jeweiligen Steuerungsmodul (310, 320, 330) entsprechender Herkunftsindikator (1, 2, ..., m) zugewiesen wird.

13. Verifizierungsverfahren (200) nach einem der Ansprüche 10 bis 12,
**dadurch gekennzeichnet, dass**
das Einlesen (203) des Verifizierungsdatensatzes (220) auf dem mindestens einen Mastersteuerungsmodul (330) implementiert ist.

14. Verifizierungsverfahren (200) nach einem der Ansprüche 10 bis 13,
**dadurch gekennzeichnet, dass**
der Verifizierungsdatensatz (220) mindestens eine vorhandene Steuerungsmodullizenz (SL_{BX}, SL_{PX}, SL_{Mx}) enthält und der Lizenzdatensatz (210) von den jeweiligen Steuerungsmodulen (310, 320, 330) verwendete Steuerungsmodullizenzen (sl_{By}, sl_{Py}, sl_{My}) enthält, wobei eine Lizenzverletzung festgestellt wird, sofern nicht jede verwendete Steuerungsmodullizenz (sl_{By}, sl_{Py}, sl_{My}) von dem Verifizierungsdatensatz (220) umfasst ist.

15. Verifizierungsverfahren (200) nach Anspruch 14,
**dadurch gekennzeichnet, dass**
jede verwendete Steuerungsmodullizenz (sl_{By}, sl_{Py}, sl_{My}) des Lizenzdatensatzes (210) mindestens zwei Parameter, einen jeweiligen Steuerungsmodultyp (B, P, M) und eine dem jeweiligen Steuerungsmodultyp (B, P, M) zugeordnete erste Steuerungsmodullizenzanzahl (y_{slB}, y_{slP}, y_{slM}) umfasst und jede vorhandene Steuerungsmodullizenz (SL_{BX}, SL_{PX}, SL_{Mx}) des Verifizierungsdatensatzes (220) mindestens zwei Parameter, einen jeweiligen Steuerungsmodultyp (B, P, M) und eine dem jeweiligen Steuerungsmodultyp (B, P, M) zugeordnete, zweite Steuerungsmodullizenzanzahl (x_{SLB}, x_{SLP}, x_{SLM}) umfasst und zum Feststellen (204) einer Lizenzverletzung mindestens ein Steuerungsmodullizenzwert (z_{SL1}, z_{SL2}, ..., z_{SLn}) jeweils einander zugeordneter Steuerungsmodultypen (B, P, M) ermittelt wird, indem die erste Steuerungsmodullizenzanzahl (y_{slB}, y_{slP}, y_{slM}) der verwendeten Steuerungsmodullizenzen (sl_{By}, sl_{Py}, sl_{my}) von der zweiten Steuerungsmodullizenzanzahl (x_{SLB}, x_{SLP}, x_{SLM}) der vorhandenen Steuerungsmodullizenzen (SL_{BX}, SL_{PX}, SL_{MX}) subtrahiert wird, und wobei eine Lizenzverletzung festgestellt wird, sofern einer der ermittelten Steuerungsmodullizenzwerte (z_{SL1}, ZSL2, ..., z_{SLn}) < 0 ist.

16. Verifizierungsverfahren (200) nach einem der vorhergehenden Ansprüche,
**dadurch gekennzeichnet, dass**
mindestens eine hinterlegte Anlagenfunktion (110, 111, 112, 113, 114) von einem Benutzer über eine Benutzeroberfläche (250) manuell auswählbar ist.

17. Programmierbares Steuerungsmodul (300) für eine Sauerstoffreduzierungsanlage (100), wobei die Sauerstoffreduzierungsanlage (100) zum Absenken und/oder Halten eines Sauerstoffkonzentrationsniveaus in mindestens einem Schutzbereich (121) durch Zuführung von aus mindestens einer Inertgasquelle (130) stammendem Inertgas (131) und zum Überwachen oder Anheben des, insbesondere zuvor abgesenkten, Sauerstoffkonzentrationsniveaus in dem Schutzbereich (121) oder einem Überwachungsbereich (122) ausgebildet ist, konfiguriert zur Durchführung eines Verifizierungsverfahrens (200) nach einem der vorhergehenden Ansprüche, mit einem oder mehreren Signaleingangskanälen (301) und einem oder mehreren Signalausgangskanälen (302) und mit mindestens einer Schnittstelle (303) zur Übertragung von Daten und/oder zur Verbindung mit einem Speichermedium (340),wobei von der Sauerstoffreduzierungsanlage (100) genutzte Anlagenfunktionen (110, 111, 112, 113, 114) anhand einer spezifischen Belegung der Signaleingangs- und Signalausgangskanäle (301, 302) erfasst werden.

18. Steuerungsmodul (300) nach Anspruch 17,
**dadurch gekennzeichnet, dass**
die Anzahl an Signaleingangskanälen (301) und/oder Signalausgangskanälen (302) durch Hinzufügen von Hardware-Komponenten (305) erweiterbar ist.

19. Sauerstoffreduzierungsanlage (100) mit mindestens einem programmierbaren Steuerungsmodul (300) nach einem der Ansprüche 17 oder 18 und geeignet zur Durchführung eines Verifizierungsverfahren (200) nach einem der Ansprüche 1 bis 16, wobei ein oder mehrere Signaleingangskanäle (301) des mindestens einen Steuerungsmoduls (300) mit Sensoren (140) der Sauerstoffreduzierungsanlage (100) und ein oder mehrere Signalausgangskanäle (302) mit Aktoren (150) der Sauerstoffreduzierungsanlage (100) verbunden sind.

## Claims

1. A verification method (200) for verifying system functions (110, 111, 112, 113, 114) of an oxygen reduction system (100), said functions being used during the operation of the oxygen reduction system (100), wherein the oxygen reduction system (100) is designed to reduce and/or maintain an oxygen concentration level in at least one protection region (121) by supplying inert gas (131) originating from at least one inert gas source (130) and to monitor or increase the oxygen concentration level, in particular previously reduced, in the protection region (121) or in a monitoring region (122), having the following method steps:
- detecting (201) system functions (110, 111, 112, 113, 114) used during the operation of the oxygen reduction system (100),
- generating (202) a license data set (210), wherein the license data set (210) contains licenses (l_{1y}, l_{2y}, ..., l_{ny}) used by the system functions (110, 111, 112, 113, 114) being used,
- reading (203) a verification data set (220) provided on a storage medium (340), wherein the verification data set (220) contains at least one existing license (L₁ₓ, L₂ₓ, ..., Lₙₓ),
- determining (204) a license violation if each license (l_{1y}, l_{2y}, ..., l_{ny}) used is not contained in the verification data set (220),
- outputting (205) a message, in particular an error message, and/or deactivating (206) at least one system function (110, 111, 112, 113, 114) if a license violation is detected, and wherein the verification method (200) is implemented on at least one programmable control module (300, 310, 320, 330) and the system functions (110, 111, 112, 113, 114) used are detected with the help of a specific assignment of signal input and signal output channels (301, 302) of the at least one programmable control module (300, 310, 320, 330).

2. The verification method (200) according to claim 1,
**characterised in that**
each license (l_{1y}, l_{2y}, ..., l_{ny}) used of the license data set (210) comprises at least two parameters: a respective license type (1, 2, ..., n) and a first number of licenses (yₗ₁, yₗ₂, ... yₗₙ) assigned to the respective license type (1, 2, ..., n), and each existing license (L₁ₓ, L₂ₓ ... Lₙₓ) of the verification data set (220) comprises at least two parameters: a respective license type (1, 2, ..., n) and a second number of licenses (x_{L1}, x_{L2}, ... x_{Ln}) assigned to the respective license type (1, 2, ..., n) and, in order to determine (204) a license violation, an in particular matrix-like assignment (230) of the licenses (l_{1y}, l_{2y}, ..., l_{ny}) used for the existing licenses (L₁ₓ, L₂ₓ ... Lₙₓ).

3. The verification method (200) according to claim 2,
**characterised in that**
in order to determine (204) a license violation, at least one license value (z₁, z₂, ..., zₙ) is determined for mutually assigned license types (1, 2, ..., n) by the first number of licenses (yₗ₁, yₗ₂, ... yₗₙ) of the licenses (l_{1y}, l_{2y}, ..., l_{ny}) used being subtracted from the second number of licenses (x_{L1}, x_{L2}, ... x_{Ln}) of the existing licenses (L₁ₓ, L₂ₓ ... Lₙₓ), and wherein a license violation is determined if one of the determined license values (z₁, z₂, ..., zₙ) is < 0.

4. The verification method (200) according to one of the preceding claims,
**characterised by**
checking (207) whether the verification data set (220) contains an activation code, wherein a message, in particular a malfunction message, is output (205) and/or at least one system function (110, 111, 112, 113, 114) is deactivated (206) if the activation code is not recognised.

5. The verification method (200) according to one of the preceding claims,
**characterised in that**
the verification data set is read in (203) via an interface (303) which is or can be connected to the storage medium (340) in a signal-transmitting manner, wherein a message, in particular a fault message, is output (205) and/or at least one system function (110, 111, 112, 113, 114) is deactivated (206) if the signal-transmitting connection between the storage medium (340) and the interface (303) is not detected.

6. The verification method (200) according to one of the preceding claims,
**characterised in that**
the verification method (200) is repeated at specific or regular time intervals, and at the same time when the message (205), in particular the error message, is output, a timer (304) is started (208) to detect the period of time during which the message, in particular the error message, is output (205).

7. The verification method (200) according to claim 6,
**characterised in that**
upon reaching or after expiry of a predetermined period of time during which the message, in particular the error message, is output (205), a fault message is output (209) and/or at least one system function (110, 111, 112, 113, 114) of the oxygen reduction system (100) is deactivated (206).

8. The verification method (200) according to one of claims 6 or 7,
**characterised in that**
upon reaching or after expiry of a predetermined period of time during which the message, in particular the fault message, is output (205), at least one system function (110, 111, 112, 113, 114) of the oxygen reduction system (100) is deactivated (206).

9. The verification method (200) according to one of the preceding claims,
**characterised in that**
the verification method (200) is implemented on several programmable and signal-connected control modules (300, 310, 320, 330), wherein at least one control module (300) is provided as a region control module (310), which is assigned to at least one protection region (121) or monitoring region (122), and/or at least one control module (300) is provided as a process control module (320), which is assigned to at least one inert gas source (130) and/or at least one control module (300) is provided as a master control module (330).

10. The verification method (200) according to claim 9,
**characterised in that**
the detecting (201) of system functions (110, 111, 112, 113, 114) used is implemented on the at least one region control module (310) and/or the at least one process control module (320) and/or the at least one master control module (330), wherein system functions (110, 111, 112, 113, 114) locally assigned to the respective control module (310, 320, 330) are detected with the help of a specific assignment of signal input and signal output channels (301, 302) of the respective region control module (310) and/or process control module (320) and/or master control module (330).

11. The verification method (200) according to one of claims 9 or 10,
**characterised by**
local generation (202) of at least one license data set (210) containing licenses (l_{1y}, l_{2y}, ..., l_{ny}) used by the respective control module (310, 320, 330), wherein each license (l_{1y}, l_{2y}, ..., l_{ny}) used there is assigned to at least one locally used system function (110, 111, 112, 113, 114) and comprises at least two parameters: a respective license type (1, 2, ..., n) and a first number of licenses (yₗ₁, yₗ₂, ... yₗₙ) assigned to the respective license type (1, 2, ..., n).

12. The verification method (200) according to claim 11,
**characterised in that**
a generating (202) of a global license data set (240) is implemented on the at least one master control module (330), and the global license data set (240) is formed by combining the locally generated license data sets (210) by the first number of licenses (yₗ₁, yₗ₂, ... yₗₙ) of the licenses (l_{1y}, l_{2y}, ..., l_{ny}) used locally by the respective control modules (310, 320, 330) being added to the mutually assigned license types (1, 2, ..., n) and wherein the licenses l_{11y}, l_{22y}, ..., l_{nmy}) used locally by the respective control modules (310, 320, 330) are assigned an origin indicator (1, 2, ..., m) corresponding to the respective control module (310, 320, 330).

13. The verification method (200) according to one of claims 10 to 12,
**characterised in that**
the reading (203) of the verification data set (220) is implemented on the at least one master control module (330).

14. The verification method (200) according to one of claims 10 to 13,
**characterised in that**
the verification data set (220) contains at least one existing control module license (SL_{BX}, SL_{Px}, SL_{MX}) and the license data set (210) contains control module licenses (sl_{By}, sl_{Py}, sl_{My}) used by the respective control modules (310, 320, 330), wherein a license violation is determined unless every control module license (sl_{By}, sl_{Py}, sl_{My}) used is included in the verification data set (220).

15. The verification method (200) according to claim 14,
**characterised in that**
each control module license (sI_{By}, sl_{Py}, sl_{My}) used of the license data set (210) comprises at least two parameters: a respective control module type (B, P, M) and a first number of control module licenses (y_{slB}, y_{slP}, y_{slM}) assigned to the respective control module type (B, P, M), and each existing control module license (SL_{BX}, SL_{PX}, SL_{MX}) of the verification data set (220) comprises at least two parameters: a respective control module type (B, P, M) and a second number of control module licenses (x_{SLB}, x_{SLP}, x_{SLM}) assigned to the respective control module type (B, P, M), and at least one control module license value (z_{SL1}, z_{SL2}, ..., z_{SLN}) of respectively mutually assigned control module types (B, P, M) is determined in order to determine (204) a license violation by the first number of control module licenses (y_{slB}, y_{slP}, y_{slM}) of the control module licenses (sl_{By}, sl_{Py}, sl_{my}) used being subtracted from the second number of control module licenses (x_{SLB}, x_{SLP}, x_{SLM}) of the existing control module licenses (SL_{BX}, SL_{PX}, SL_{Mx}), and wherein a license violation is determined if one of the determined control module license values (z_{SL1}, z_{SL2}, ..., z_{SLn}) is < 0.

16. The verification method (200) according to one of the preceding claims,
**characterised in that**
at least one stored system function (110, 111, 112, 113, 114) can be selected manually by a user via a user interface (250).

17. A programmable control module (300) for an oxygen reduction system (100), wherein the oxygen reduction system (100) is designed to lower and/or maintain an oxygen concentration level in at least one protection region (121) by supplying inert gas (131) from at least one inert gas source (130) and to monitor or increase the oxygen concentration level, in particular previously reduced, in the protection region (121) or a monitoring region (122), and is configured to carry out a verification method (200) according to one of the preceding claims, with one or more signal input channels (301) and one or more signal output channels (302) and with at least one interface (303) for transmitting data and/or for connection to a storage medium (340), wherein system functions (110, 111, 112, 113, 114) used by the oxygen reduction system (100) are detected with the help of a specific assignment of signal input and signal output channels (301, 302).

18. The control module (300) according to claim 17,
**characterised in that**
the number of signal input channels (301) and/or signal output channels (302) can be expanded by adding hardware components (305).

19. An oxygen reduction system (100) with at least one programmable control module (300) according to one of claims 17 or 18 and suitable for carrying out a verification method (200) according to one of claims 1 to 16, wherein one or more signal input channels (301) of the at least one control module (300) are connected to sensors (140) of the oxygen reduction system (100) and one or more signal output channels (302) are connected to actuators (150) of the oxygen reduction system (100).

## Revendications

1. Procédé de vérification (200) pour vérifier, dans une installation de réduction d'oxygène en fonctionnement (100), les fonctions d'installation utilisées (110, 111, 112, 113, 114) de l'installation de réduction d'oxygène (100), procédé dans lequel l'installation de réduction d'oxygène (100) est conformée pour abaisser et/ou maintenir un niveau de concentration en oxygène dans au moins une zone de protection (121) par la fourniture de gaz inerte (130) provenant d'au moins une source de gaz inerte (131) et pour contrôler ou augmenter le niveau de concentration d'oxygène, en particulier préalablement abaissé, dans la zone de protection (121) ou dans une zone de surveillance (122), lequel procédé comprenant les étapes de procédé suivantes :
- détection (201) de données relatives aux fonctions d'installation utilisées (110, 111, 112, 113, 114) lors du fonctionnement de l'installation de réduction d'oxygène (100),
- création (202) d'un ensemble de données d'autorisation (210), l'ensemble de données d'autorisation (210) contenant des autorisations (l_{1y}, l_{2y}, ..., l_{ny}) utilisées par les fonctions d'installation utilisées (110, 111, 112, 113, 114),
- lecture (203) d'un ensemble de données de vérification (220) mis à disposition par un support de stockage (340), l'ensemble de données de vérification (220) contenant au moins une autorisation existante (L₁ₓ, L₂ₓ ... Lₙₓ),
- détermination (204) d'une violation d'autorisation si l'ensemble de données de vérification (220) ne contient pas chaque autorisation utilisée (l_{1y}, l_{2y} ..., Iny),
- émission (205) d'un message, en particulier d'un message d'erreur et/ou désactivation (206) d'au moins une fonction d'installation (110, 111, 112, 113, 114), dans le cas où une violation d'autorisation est constatée, et dans lequel le procédé de vérification (200) est implémenté sur au moins un module de commande programmable (300, 310, 320, 330) et les fonctions d'installation utilisées (110, 111, 112, 113, 114) sont détectées à partir d'une occupation spécifique de canaux d'entrée de signaux et de canaux de sortie de signaux (301, 302) dudit au moins un module de commande programmable (300, 310, 320, 330).

2. Procédé de vérification (200) selon la revendication 1, **caractérisé en ce que** chaque autorisation utilisée (l_{1y}, l_{2y}, ... , l_{ny}) de l'ensemble de données d'autorisation (210) comprend au moins deux paramètres, un type d'autorisation respectif (1, 2, ... , n) et un premier nombre d'autorisation (yₗ₁, yₗ₂, ... yₗₙ) associé au type d'autorisation respectif (1, 2, ... , n), et chaque autorisation existante (L₁ₓ, L₂ₓ ... Lₙₓ) de l'ensemble de données de vérification (220) comprend au moins deux paramètres, un type d'autorisation respectif (1, 2, ..., n) et un deuxième nombre d'autorisation (x_{L1}, x_{L2}, ... x_{Ln}) associé au type d'autorisation respectif (1, 2, ..., n), et **en ce que**, pour constater (204) une violation d'autorisation, on procède à une affectation (230), en particulier matricielle, des autorisations utilisées (l_{1y}, l_{2y}, ... , l_{ny}) aux autorisations existantes (L₁ₓ, L₂ₓ ... Lₙₓ).

3. Procédé de vérification (200) selon la revendication 2,
**caractérisé en ce que**
pour constater (204) une violation d'autorisation, on détermine au moins une valeur d'autorisation (z₁, z₂, ... , zₙ), lesquelles sont associées à des types d'autorisation respectifs (1, 2, ... , n), en soustrayant le premier nombre d'autorisation (yₗ₁, yₗ₂, ... yₗₙ) des autorisations utilisées (l_{1y}, l_{2y}, ... , l_{ny}) du deuxième nombre d'autorisation (x_{L1}, x_{L2}, ... x_{Ln}) des autorisations existantes (L₁ₓ, L₂ₓ ... Lₙₓ), et on constate une violation d'autorisation si l'une des valeurs d'autorisation déterminées (z₁, z₂, ... , zₙ) est < 0.

4. Procédé de vérification (200) selon l'une quelconque des revendications précédentes,
**caractérisé par**
on vérifie (207) si l'enregistrement de vérification (220) contient un code de déverrouillage, et on émet (205) alors un message, en particulier un message de dysfonctionnement, et/ou on désactive (206) au moins une fonction d'installation (110, 111, 112, 113, 114) dans le cas où le code de déverrouillage n'est pas reconnu.

5. Procédé de vérification (200) selon l'une quelconque des revendications précédentes,
**caractérisé en ce que**
on réalise la lecture (203) de l'enregistrement de vérification par l'intermédiaire d'une interface (303) reliée ou pouvant être reliée au support de stockage (340) par transmission de signaux, et on émet (205) un message, en particulier un message de dysfonctionnement, et/ou on désactive (206) au moins une fonction d'installation (110, 111, 112, 113, 114) dans le cas où la liaison de transmission de signaux entre le support de stockage (340) et l'interface (303) n'est pas reconnue.

6. Procédé de vérification (200) selon l'une quelconque des revendications précédentes,
**caractérisé en ce que**
on répète le procédé de vérification (200) à intervalles déterminés ou réguliers, et en même temps que l'envoi du message (205), en particulier du message d'erreur, on lance (208) un chronomètre (304) pour enregistrer la durée pendant laquelle le message, en particulier le message d'erreur, est émis (205).

7. Procédé de vérification (200) selon la revendication 6,
**caractérisé en ce que**
à l'atteinte ou après l'expiration d'un laps de temps prédéfini pendant lequel est émis (205) le message, en particulier le message d'erreur, on émet (209) un message de dysfonctionnement et/ou on désactive (206) au moins une fonction d'installation (110, 111, 112, 113, 114) de l'installation de réduction d'oxygène (100).

8. Procédé de vérification (200) selon l'une des revendications 6 ou 7,
**caractérisé en ce que**
à l'atteinte ou après l'expiration d'un laps de temps prédéfini pendant lequel est émis (205) le message, en particulier le message de dysfonctionnement, une désactivation (206) d'au moins une fonction d'installation (110, 111, 112, 113, 114) de l'installation de réduction d'oxygène (100) a lieu.

9. Procédé de vérification (200) selon l'une quelconque des revendications précédentes,
**caractérisé en ce que**
le procédé de vérification (200) est implémenté sur plusieurs modules de commande (300, 310, 320, 330) programmables et reliés entre eux par des signaux, dans lesquels on prévoit qu'au moins un module de commande (300) sert de module de commande de zone (310), qui correspond à au moins une zone de protection (121) ou une zone de surveillance (122), et/ou qu'au moins un module de commande (300) sert de module de commande de processus (320), qui est associé à au moins une source de gaz inerte (130), et/ou qu'au moins un module de commande (300) sert de module de commande maître (330).

10. Procédé de vérification (200) selon la revendication 9,
**caractérisé en ce que**
la détection (201) de fonctions d'installation (110, 111, 112, 113, 114) utilisées est mise en oeuvre sur ledit au moins un module de commande de zone (310) et/ou sur ledit au moins un module de commande de processus (320) et/ou sur ledit au moins un module de commande maître (330), dans laquelle les fonctions d'installation (110, 111, 112, 113, 114) attribuées localement au module de commande (310, 320, 330) respectif sont détectées à l'aide d'une occupation spécifique de canaux d'entrée de signaux et de canaux de sortie de signaux (301, 302) du module de commande de zone (310) et/ou du module de commande de processus (320) et/ou du module de commande maître (330) respectif.

11. Procédé de vérification (200) selon l'une des revendications 9 ou 10,
**caractérisé par**
la création locale (202) d'au moins un ensemble de données d'autorisation (210) contenant les autorisations (l_{1y}, l_{2y}, ... , l_{ny}) utilisées par le module de commande (310, 320, 330) concerné, dans laquelle chaque autorisation (l_{1y}, l_{2y}, ... , l_{ny}) utilisée dans cet ensemble de données est associée à au moins une fonction d'installation (110, 111, 112, 113, 114) utilisée localement et comprend au moins deux paramètres, un type d'autorisation respectif (1, 2, ..., n) et un premier nombre d'autorisation (yₗ₁, yₗ₂, ... yₗₙ) associé au type d'autorisation respectif (1, 2, ..., n).

12. Procédé de vérification (200) selon la revendication 11,
**caractérisé en ce que**
une création (202) d'un ensemble global de données d'autorisation (240) est implémentée sur ledit au moins un module de commande maître (330), et l'ensemble global de données d'autorisation (240) est formé par assemblage des ensembles de données d'autorisation (210) créés localement, **en ce qu'**on additionne les premiers nombres d'autorisation (yₗ₁, yₗ₂, ... yₗₙ) des autorisations (l_{1y}, l_{2y}, ... , l_{ny}) utilisées localement par les modules de commande respectifs (310, 320, 330) et associés aux types d'autorisation respectifs (1, 2, ... , n), et **en ce qu'**on attribue aux autorisations (l_{11y}, l_{22y}, ... , l_{nmy}) utilisées localement par les modules de commande respectifs (310, 320, 330) un indicateur d'origine (1, 2, ..., m) correspondant au module de commande respectif (310, 320, 330).

13. Procédé de vérification (200) selon l'une des revendications 10 à 12,
**caractérisé en ce que**
la lecture (203) de l'ensemble de données de vérification (220) est implémentée sur ledit au moins un module de commande maître (330).

14. Procédé de vérification (200) selon l'une quelconque des revendications 10 à 13,
**caractérisé en ce que**
l'ensemble de données de vérification (220) contient au moins une autorisation existante de module de commande (SL_{BX}, SL_{Px}, SL_{Mx}) et l'ensemble de données d'autorisation (210) contient les autorisations de module de commande (sl_{By}, sl_{Py}, sl_{My}) utilisées par les modules de commande respectifs (310, 320, 330), une violation d'autorisation étant constatée si l'ensemble de données de vérification (220) ne contient pas chaque autorisation de module de commande (sl_{By}, sl_{Py}, sl_{My}) utilisée.

15. Procédé de vérification (200) selon la revendication 14,
**caractérisé en ce que**
chaque autorisation de module de commande (sl_{By}, sl_{Py}, sl_{My}) utilisée de l'ensemble de données d'autorisation (210) comprend au moins deux paramètres, un type de module de commande respectif (B, P, M) et un premier nombre d'autorisation de module de commande (y_{slB}, y_{slP}, y_{slM}) correspondant à ce type de module de commande (B, P, M) respectif, et chaque autorisation existante de module de commande (SL_{BX}, SL_{PX}, SL_{Mx}) de l'ensemble de données de vérification (220) comprend au moins deux paramètres, un type de module de commande respectif (B, P, M) et un deuxième nombre d'autorisation de module de commande (x_{SLB}, x_{SLP}, x_{SLM}) associé à ce type de module de commande respectif (B, P, M), et, pour constater (204) une violation d'autorisation, on détermine au moins une des valeurs d'autorisation de module de commande (z_{SL1}, z_{SL2}, ... , z_{SLn}) qui sont associées chacune à un type de module de commande (B, P, M) respectif, pour cela le premier nombre d'autorisation de module de commande (y_{slB}, y_{slP}, y_{slM}) des autorisations de module de commande (sl_{By}, sl_{Py}, sl_{My}) utilisées est soustrait du deuxième nombre d'autorisation de module de commande (x_{SLB}, x_{SLP}, x_{SLM}) des autorisations existantes de module de commande (SL_{BX}, SL_{PX}, SL_{Mx}), et dans lequel une violation d'autorisation est constatée dans le cas où l'une des valeurs d'autorisation de module de commande (z_{SL1}, z_{SL2}, ... , z_{SLn}) est < 0.

16. Procédé de vérification (200) selon l'une quelconque des revendications précédentes,
**caractérisé en ce que**
au moins une fonction d'installation choisie (110, 111, 112, 113, 114) peut être sélectionnée manuellement par un utilisateur via une interface utilisateur (250).

17. Module de commande programmable (300) pour une installation de réduction d'oxygène (100), dans lequel l'installation de réduction d'oxygène (100) est conformée pour abaisser et/ou maintenir un niveau de concentration d'oxygène dans au moins une zone de protection (121) en amenant du gaz inerte (131) provenant d'au moins une source de gaz inerte (130) et pour surveiller ou augmenter le niveau de concentration d'oxygène, en particulier préalablement abaissé, dans la zone de protection (121) ou une zone de surveillance (122), module configuré pour la mise en oeuvre d'un procédé de vérification (200) selon l'une quelconque des revendications précédentes, avec un ou plusieurs canaux d'entrée de signaux (301) et un ou plusieurs canaux de sortie de signaux (302) et avec au moins une interface (303) pour la transmission de données et/ou pour la connexion à un support de stockage (340), dans lequel des fonctions d'installation (110, 111, 112, 113, 114) utilisées par l'installation de réduction d'oxygène (100) sont détectées à partir d'une occupation spécifique des canaux d'entrée de signaux et des canaux de sortie de signaux (301, 302).

18. Module de commande (300) selon la revendication 17,
**caractérisé en ce que**
le nombre de canaux d'entrée de signaux (301) et/ou de canaux de sortie de signaux (302) peut être étendu par l'ajout de composants matériels (305).

19. Installation de réduction d'oxygène (100) comprenant au moins un module de commande programmable (300) selon l'une des revendications 17 ou 18 et apte à mettre en oeuvre un procédé de vérification (200) selon l'une quelconque des revendications 1 à 16, dans lequel un ou plusieurs canaux d'entrée de signaux (301) dudit au moins un module de contrôle (300) sont connectés avec des capteurs (140) de l'installation de réduction d'oxygène (100), et un ou plusieurs canaux de sortie de signaux (302) sont connectés avec des actionneurs (150) de l'installation de réduction d'oxygène (100).
